(19) Europäisches Patentamt
European Patent Office
Office européen des brevets

(11) Publication number: 0 518 216 A2

# (12) EUROPEAN PATENT APPLICATION

(21) Application number: **92109470.2**

(51) Int. Cl.5: **C07D 295/12**, A61K 31/395

(22) Date of filing: **04.06.92**

(30) Priority: **06.06.91 US 711234**

(43) Date of publication of application:
**16.12.92 Bulletin 92/51**

(84) Designated Contracting States:
**PT**

(71) Applicant: **G.D. Searle & Co.**
**P.O. Box 5110**
**Chicago Illinois 60680(US)**

(72) Inventor: **De Costa, Brian R.**
**8232 Emory Grove Road**
**Gaithersburg, Maryland 20877(US)**
Inventor: **Radesca, Lilian**
**203 Cheltenham Road**
**Newark, Delaware 19711(US)**
Inventor: **Bowen, Wayne**
**7615 Nutwood Court**
**Derwood, Maryland 20855(US)**
Inventor: **Long, Joseph**
**13833 Lakeside Drive**
**Clarksville, Maryland 21029(US)**
Inventor: **Walker, J. Michael**
**131 Betsy Williams Drive**
**Cranston, Rhode Island 02912(US)**
Inventor: **Rice, Kenneth C.**
**9007 Kirkdale Road**
**Bethesda, Maryland 20817(US)**
Inventor: **Gray, Nancy M.**
**1182 Sandhurst Drive**
**Buffalo Grove, Illinois 60089(US)**
Inventor: **Contreras, Patricia C.**
**3500 Church Street**
**Evanston, Illinois 60203(US)**

(74) Representative: **Beil, Hans Chr., Dr. et al**
**Beil, Wolff und Beil, Rechtsanwälte**
**Adelonstrasse 58 Postfach 80 01 40**
**W-6230 Frankfurt am Main 80(DE)**

## (54) N-/Arylethyl/-N-alkyl-2-(1-pyrrolidinyl)ethylamine derivatives for CNS disorders.

(57) Certain N-[arylethyl]-N-alkyl-2-(1-pyrrolidinyl)ethylamine compounds are described for treatment of CNS disorders such as cerebral ischemia, psychotic disorders, convulsions and parkinsonism. Compounds of particular interest are of the formula

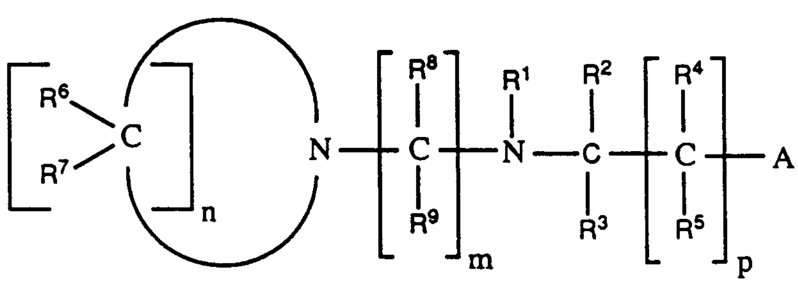

wherein $R^1$ is selected from hydrido, loweralkyl, cycloalkylalkyl of four to six carbon atoms and loweralkenyl-loweralkyl; wherein each of $R^2$ and $R^3$ is independently selected from hydrido and loweralkyl; wherein each of $R^4$ through $R^9$ is independently selected from hydrido, hydroxy, loweralkyl, benzyl, phenoxy, benzyloxy and haloloweralkyl; wherein m is a number selected from one through three; wherein n is a number selected from four through seven; wherein p is a number selected from zero through four; wherein A is selected from phenyl, naphthyl and thienyl; wherein any of the foregoing A groups can be further substituted with one or more

EP 0 518 216 A2

substituents independently selected from hydrido, hydroxy, loweralkyl, loweralkoxy, halo, haloloweralkyl, amino, monoloweralkylamino and diloweralkylamino; or a pharmaceutically acceptable salt thereof.

## Field of the Invention

This invention is in the field of clinical neurology and relates specifically to a class of therapeutically useful compounds, compositions and methods for treatment of Central Nervous System (CNS) dysfunctions, neurotoxic damage, or neurodegenerative diseases. For example, these compounds are particularly useful for treating neurotoxic injury which follows periods of hypoxia, anoxia or ischemia associated with stroke, cardiac arrest or perinatal asphyxia. These compounds are also useful as antipsychotics and anticonvulsives.

## Background of the Invention

Unlike other tissues which can survive extended periods of hypoxia, brain tissue is particularly sensitive to deprivation of oxygen or energy. Permanent damage to neurons can occur during brief periods of hypoxia, anoxia or ischemia. Neurotoxic injury is known to be caused or accelerated by certain excitatory amino acids (EAA) found naturally in the central nervous system (CNS). Glutamate (Glu) is an endogenous amino acid which has been characterized as a fast excitatory transmitter in the mammalian brain. Glutamate is also known as a powerful neurotoxin capable of killing CNS neurons under certain pathological conditions which accompany stroke and cardiac arrest. Normal glutamate concentrations are maintained within brain tissue by energy-consuming transport systems. Under low energy conditions which occur during conditions of hypoglycemia, hypoxia or ischemia, cells can release glutamate. Under such low energy conditions the cell is not able to take glutamate back into the cell. Initial glutamate release stimulates further release of glutamate which results in an extracellular glutamate accumulation and a cascade of neurotoxic injury.

It has been shown that the sensitivity of central neurons to hypoxia and ischemia can be reduced by either blockage of synaptic transmission or by the specific antagonism of postsynaptic glutamate receptors [see S. M. Rothman and J. W. Olney, "Glutamate and the Pathophysiology of Hypoxia - Ischemic Brain Damage," Annals of Neurology, Vol. 19, No. 2 (1986)]. Glutamate is characterized as a broad spectrum agonist having activity at three neuronal excitatory amino acid receptor sites. These receptor sites are named after the amino acids which selectively excite them, namely: Kainate (KA), N-methyl-D-aspartate (NMDA or NMA) and quisqualate (QUIS).

Neurons which have EAA receptors on their dendritic or somal surfaces undergo acute excitotoxic degeneration when these receptors are excessively activated by glutamate. Thus, agents which selectively block or antagonize the action of glutamate at the EAA synaptic receptors of central neurons can prevent neurotoxic injury associated with hypoxia, anoxia, or ischemia caused by stroke, cardiac arrest or perinatal asphyxia.

It is known that compounds of various structures, such aminophosphonovalerate derivatives and piperidine dicarboxylate derivatives, may act as competitive antagonists at the NMDA receptor. Certain piperidineethanol derivatives, such as ifenprodil and 1-(4-chlorophenyl)-2-[1-(4-fluorophenyl)piperidinyl]-ethanol, which are known anti-ischemic agents, have been found to be non-competitive NMDA receptor antagonists [C. Carter et al, J. Pharm Exp. Ther., 247 (3), 1222-1232 (1988)].

There are many classes of compunds known for treatment of psychotic disorders. For example, current therapeutic treatments for psychoses use compounds classifiable as phenothiazine-thioxanthenes, as phenylbutylpiperidines and also as certain alkaloids. An example of a phenylbutylpiperidine compound of current use in psychotic treatment therapy is haloperidol [A.F. Gilman et al, The Pharmacological Basis of Therapeutics, 7th Edn., p. 404, MacMillan (1985)].

Certain nitrogen-containing cyclohetero cycloalkylaminoaryl compounds are known for pharmaceutical purposes. For example, U.S. Patent No. 4,204,003 to Szmuszkovicz describes N-(2-aminocyclopentyl)-N-alkanoylanilides as antidepressant agents.

Certain aminocycloaliphatic benzamides have been described for various uses. For example, U.S. Patent No. 4,463,013 to Collins et al describes aminocyclohexylbenzamides for use as diuretic agents. The compound (±)-trans-3,4-dichloro-N-methy-N-[2-(1-pyrrolidinyl)cyclohexyl]benzene-acetamide has been evaluated for its selectivity as an amino acid antagonist [C.G. Parsons et al, Neuropharm., 25(2), 217-220 (1986)]. This same compound has been evaluated for its neuroprotective activity against kainate-induced toxicity [W. Lason et al, Brain Res., 482, 333-339 (1989)]. U.S. Patent No. 4,801,604 to Vonvoightlander et al describes certain cis-N-(2-aminocycloaliphatic)benzamides as anticonvulsants including, specifically, the compound cis-3,4-dichloro-N-methyl-N-[2-(1-pyrrolidinyl)cyclohexyl]benzamide. These benzeneacetamide derivatives, such as trans-3,4-dichloro-N-methyl-N-[2-(1-pyrrolidinyl)cyclohexyl]-benzeneacetamide, have been described as a highly selective ligand for kappa opioid receptors. Such kappa opioid affinity is believed associated with blockade of convulsions and protection from cerebral ischemia [B.R. de Costa et

al, J. Med. Chem., 32(8), 1996-2002 (1989)].

Description of the Invention

Treatment of CNS disorders and diseases such as cerebral ischemia, psychotic disorders, convulsions and parkinsonism, as well as prevention of neurotoxic damage and neurodegenerative diseases, may be accomplished by administration of a therapeutically-effective amount of a compound of Formula I:

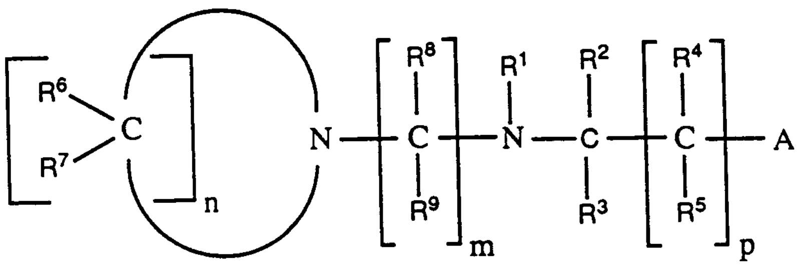

(I)

wherein $R^1$ is selected from hydrido, alkyl, cycloalkyl, hydroxyalkyl, haloalkyl, cycloalkylalkyl, alkoxyalkyl, aralkyl, aryl, alkenylalkyl, alkynylalkyl, carboxyalkyl, alkanoyl, alkylsulfinyl, alkylsulfonyl, arylsulfinyl and arylsulfonyl; wherein each of $R^2$ and $R^3$ is independently selected from hydrido, alkyl, cycloalkyl, hydroxyalkyl, haloalkyl, cycloalkylalkyl, alkoxyalkyl, aralkyl, aryl, alkenyl, alkynyl, alkenylalkyl, alkynylalkyl, carboxyalkyl, alkanoyl, alkoxycarbonyl, carboxy, cyanoalkyl, alkylsulfinyl, alkylsulfonyl, arylsulfinyl and arylsulfonyl; wherein each of $R^4$ through $R^9$ is independently selected from hydrido, hydroxy, alkyl, cycloalkyl, cycloalkylalkyl, aralkyl, aryl, alkoxy, aryloxy, aralkoxy, alkoxyalkyl, haloalkyl, hydroxyalkyl, cyano, amino, monoalkylamino, dialkylamino, carboxy, carboxyalkyl, alkanoyl, alkenyl and alkynyl; wherein $R^4$ and $R^5$ may be taken together to form oxo; wherein $R^6$ and $R^7$ may be taken together to form oxo; wherein $R^8$ and $R^9$ may be taken together to form oxo; wherein m is a number selected from one through five; wherein n is a number selected from four through nine; wherein p is a number selected from zero through five; wherein A is selected from aryl, heteroaryl, aryloxy, heteroaryloxy, aralkoxy, heteroaralkoxy, arylamino, heteroarylamino, aralkylamino, heteroaralkylamino, arylthio, heteroarylthio, aralkylthio and heteroaralkylthio; wherein any of the foregoing A groups can be further substituted with one or more substituents independently selected from hydrido, hydroxy, alkyl, cycloalkyl, cycloalkylalkyl, aralkyl, aryl, alkoxy, aryloxy, aralkoxy, alkoxyalkyl, halo, haloalkyl, hydroxyalkyl, cyano, amino, monoalkylamino, dialkylamino, carboxy, carboxyalkyl, alkanoyl, alkenyl and alkynyl; or a pharmaceutically-acceptable salt thereof.

A preferred family of compounds of Formula I consists of those compounds wherein $R^1$ is selected from hydrido, alkyl, cycloalkyl, hydroxyalkyl, haloalkyl, cycloalkylalkyl, alkoxyalkyl, aralkyl, aryl, alkenylalkyl, alkynylalkyl and carboxyalkyl; wherein each of $R^2$ and $R^3$ is independently selected from hydrido, alkyl, cycloalkyl, hydroxyalkyl, haloalkyl, cycloalkylalkyl, alkoxyalkyl, aralkyl, aryl, alkenyl, alkynyl, alkenylalkyl, alkynylalkyl, carboxyalkyl, alkanoyl, alkoxycarbonyl, carboxy and cyanoalkyl; wherein each of $R^4$ through $R^9$ is independently selected from hydrido, hydroxy, alkyl, cycloalkyl, cycloalkylalkyl, aralkyl, aryl, alkoxy, aryloxy, aralkoxy, alkoxyalkyl, haloalkyl, hydroxyalkyl, cyano, amino, monoalkylamino, dialkylamino, carboxy, carboxyalkyl, alkanoyl, alkenyl and alkynyl; wherein $R^4$ and $R^5$ may be taken together to form oxo; wherein m is a number selected from one through four; wherein p is a number selected from zero through four;

wherein A is selected from aryl, heteroaryl, aryloxy, heteroaryloxy, aralkoxy, heteroaralkoxy, arylamino, heteroarylamino, aralkylamino, heteroaralkylamino, arylthio, heteroarylthio, aralkylthio and heteroaralkylthio; wherein any of the foregoing A groups can be further substituted with one or more substituents independently selected from hydrido, hydroxy, alkyl, cycloalkyl, cycloalkylalkyl, aralkyl, aryl, alkoxy, aryloxy, aralkoxy, alkoxyalkyl, halo, haloalkyl, hydroxyalkyl, cyano, amino, monoalkylamino, dialkylamino, carboxy, carboxyalkyl, alkanoyl, alkenyl and alkynyl; or a pharmaceutically acceptable salt thereof.

A more preferred family of compounds within Formula I consists of those compounds wherein $R^1$ is selected from hydrido, loweralkyl, cycloalkyl of three to about eight carbon atoms, hydroxyloweralkyl, haloloweralkyl, cycloalkylalkyl of four to about eight carbon atoms, loweralkoxyloweralkyl, phenylloweralkyl, phenyl, loweralkenylloweralkyl, loweralkynylloweralkyl and carboxyloweralkyl; wherein each of $R^2$ and $R^3$ is independently selected from hydrido, loweralkyl, cycloalkyl of three to about eight carbon atoms, hydroxyloweralkyl, haloloweralkyl, cycloalkylalkyl of four to about eight carbon atoms, loweralkoxyloweralkyl,

phenylloweralkyl, phenyl, loweralkenyl, loweralkynyl, loweralkenylloweralkyl, loweralkynylloweralkyl, carboxyloweralkyl, loweralkanoyl, loweralkoxycarbonyl, carboxy and cyanoloweralkyl; wherein each of $R^4$ through $R^9$ is independently selected from hydrido, hydroxy, loweralkyl, cycloalkyl of three to about eight carbon atoms, cycloalkylalkyl of four to about eight carbon atoms, phenylloweralkyl, phenyl, loweralkoxy, phenoxy, phenylloweralkoxy, loweralkoxyloweralkyl, haloloweralkyl, hydroxyloweralkyl, cyano, amino, monoloweralkylamino, diloweralkylamino, carboxy, carboxyloweralkyl, loweralkanoyl, loweralkenyl and loweralkynyl; wherein $R^4$ and $R^5$ may be taken together to form oxo; wherein m is a number selected from one through four; wherein n is a number selected from four through eight; wherein p is a number selected from zero through three; wherein A is selected from phenyl, naphthyl, heteroaryl, phenoxy, naphthyloxy, heteroaryloxy, phenylloweralkoxy, naphthylloweralkoxy, heteroarylloweralkoxy, phenylamino, naphthylamino, heteroarylamino, phenylloweralkylamino, naphthylloweralkylamino, heteroaralkylamino, phenylthio, naphthylthio, heteroarylthio, phenylloweralkylthio and heteroarylloweralkylthio; wherein any of the foregoing A groups can be further substituted with one or more substituents independently selected from hydrido, hydroxy, loweralkyl, cycloalkyl of three to about eight carbon atoms, cycloalkylalkyl of four to about eight carbon atoms, phenylloweralkyl, phenyl, loweralkoxy, phenoxy, phenyloweralkoxy, loweralkoxyloweralkyl, halo, haloloweralkyl, hydroxyloweralkyl, cyano, amino, monoloweralkylamino, diloweralkylamino, carboxy, carboxyloweralkyl, loweralkanoyl, loweralkenyl and loweralkynyl; or a pharmaceutically acceptable salt thereof.

A more highly preferred family of compounds of Formula I consists of those compounds wherein $R^1$ is selected from hydrido, loweralkyl, cycloalkyl of three to about eight carbon atoms, cycloalkylalkyl of four to about eight carbon atoms, benzyl, phenyl, loweralkenylloweralkyl and loweralkynylloweralkyl; wherein each of $R^2$ and $R^3$ is independently selected from hydrido, loweralkyl, cycloalkyl of three to about eight carbon atoms, cycloalkylalkyl of four to about eight carbon atoms, benzyl, phenyl, loweralkenyl, loweralkynyl, loweralkenylloweralkyl, loweralkynylloweralkyl, loweralkanoyl and loweralkoxycarbonyl; wherein each of $R^4$ through $R^9$ is independently selected from hydrido, hydroxy, loweralkyl, cycloalkyl of three to about eight carbon atoms, cycloalkylalkyl of four to about eight carbon atoms, benzyl, phenyl, loweralkoxy, phenoxy, benzyloxy, loweralkoxyloweralkyl, haloloweralkyl, hydroxyloweralkyl, loweralkanoyl, loweralkenyl and loweralkynyl; wherein $R^4$ and $R^5$ may be taken together to form oxo; wherein m is a number selected from one through three; wherein n is a number selected from four through seven; wherein p is a number selected from zero through three; wherein A is selected from phenyl, naphthyl, thienyl, phenoxy, benzyloxy, naphthyloxy, thiophenoxy, phenylamino, benzylamino, naphthylamino, phenylthio, benzylthio and naphthylthio; wherein any of the foregoing A groups can be further substituted with one or more substituents independently selected from hydrido, hydroxy, loweralkyl, cycloalkyl of three to about eight carbon atoms, cycloalkylalkyl of four to about eight carbon atoms, loweralkoxy, loweralkoxyloweralkyl, halo, haloloweralkyl, hydroxyloweralkyl, amino, monoloweralkylamino, diloweralkylamino, loweralkanoyl, loweralkenyl and loweralkynyl; or a pharmaceutically acceptable salt thereof.

An even more highly preferred family of comopunds of Formula I consists of those compounds wherein $R^1$ is selected from hydrido, loweralkyl, cycloalkylalkyl of four to six carbon atoms and loweralkenylloweralkyl; wherein each of $R^2$ and $R^3$ is independently selected from hydrido and loweralkyl; wherein each of $R^4$ through $R^9$ is independently selected from hydrido, hydroxy, loweralkyl, benzyl, phenoxy, benzyloxy and haloloweralkyl; wherein m is a number selected from one through three; wherein n is a number selected from four through six; wherein p is a number selected from zero through two; wherein A is selected from phenyl, naphthyl and thienyl; wherein any of the foregoing A groups can be further substituted with one or more substituents independently selected from hydrido, hydroxy, loweralkyl, loweralkoxy, halo, haloloweralkyl, amino, monoloweralkylamino and diloweralkylamino; or a pharmaceutically acceptable salt thereof.

A most preferred family of compounds within Formula I consists of compounds wherein $R^1$ is selected from hydrido, methyl, ethyl, propyl, cyclopropylmethyl, allyl and dimethylallyl; wherein each of $R^2$ and $R^3$ is independently selected from hydrido, methyl, ethyl and propyl; wherein each of $R^4$ through $R^9$ is independently selected from hydrido, hydroxy, methyl, ethyl, propyl, benzyl, phenoxy, benzyloxy and haloloweralkyl; wherein m is a number selected from one through three; wherein n is a number selected from four through six; wherein p is a number selected from zero or one; wherein A is phenyl or naphthyl; wherein any of the foregoing A groups can be further substituted with one or more substituents independently selected from hydroxy, methyl, ethyl, propyl, methoxy, ethoxy, methylenedioxy, halo, trifluoromethyl, amino, methylamino and dimethylamino; or a pharmaceutically acceptable salt thereof.

The term "hydrido" denotes a single hydrogen atom (H). This hydrido group may be attached, for example, to an oxygen atom to form a hydroxyl group; or as another example, two hydrido groups may be attached to a carbon atom to form a divalent $-CH_2-$ group, that is, a "methylene" group; or as another example, one hydrido group may be attached to a carbon atom to form a trivalent $-CH<$ group. Where the term "alkyl" is used, either alone or within other terms such as "haloalkyl" and "hydroxyalkyl", the term

"alkyl" embraces linear or branched radicals having one to about twenty carbon atoms or, preferably, one to about ten carbon atoms. More preferred alkyl radicals are "lower alkyl" radicals having one to about five carbon atoms. The term "cycloalkyl" embraces cyclic radicals having three to about six carbon atoms, such as cyclopropyl and cyclobutyl. The term "haloalkyl" embraces radicals wherein any one or more of the alkyl carbon atoms is substituted with one or more halo groups, preferable selected from bromo, chloro and fluoro. Specifically embraced by the term "haloalkyl" are monohaloalkyl, dihaloalkyl and polyhaloalkyl groups. A monohaloalkyl group, for example, may have either a bromo, a chloro, or a fluoro atom within the group. Dihaloalkyl and polyhaloalkyl groups may be substituted with two or more of the same halo groups, or may have a combination of different halo groups. A dihaloalkyl group, for example, may have two bromo atoms, such as a dibromomethyl group, or two chloro atoms, such as a dichloromethyl group, or one bromo atom and one chloro atom, such as a bromochloromethyl group. An example of a polyhaloalkyl is a trifluoromethyl group. The terms "alkylol" and "hydroxyalkyl" embrace linear or branched alkyl groups having one to about ten carbon atoms any one of which may be substituted with one or more hydroxyl groups. The term "alkenyl" embraces linear or branched radicals having two to about twenty carbon atoms, preferable two to about ten carbon atoms, and containing at least one carbon-carbon triple bond. The terms "cycloalkenyl" and "cycloalkynyl" embrace cyclic radicals having three to about ten ring carbon atoms including, respectively, one or more double or triple bonds involving adjacent ring carbons. The terms "alkoxy" and "alkoxyalkyl" embrace linear or branched oxy-containing radicals each having alkyl portions of one to about ten carbon atoms, such as methoxy group. The "alkoxy" or "alkoxyalkyl" radicals may be further substituted with one or more halo atoms, such as fluoro, chloro or bromo, to provide haloalkoxy or haloalkoxyalkyl groups. The term "heteroaryl" embraces aromatic ring systems containing one or two hetero atoms selected from oxygen, nitrogen and sulfur in a ring system having five or six ring members, examples of which are thienyl, furanyl, pyridinyl, thiazolyl, pyrimidyl and isoxazolyl. The term "alkylene chain" describes a chain of two to six methylene ($-CH_2-$) groups which may form a cyclic structure with or without a hetero atom in the cyclic structure.

Specific examples of alkyl groups are methyl, ethyl, n-propyl, isopropyl, n-butyl, sec-butyl, iso-butyl, tert-butyl, n-pentyl, iso-pentyl, methyl-butyl, dimethylbutyl and neopentyl. Typical alkenyl and alkynyl groups may have one unsaturated bond, such as an allyl group, or may have a plurality of unsaturated bonds, with such plurality of bonds either adjacent, such as allene-type structures, or in conjugation, or separated by several saturated carbons.

Included within the family of compounds of Formula I are the tautomeric forms of the described compounds, isomeric forms including diastereoisomers, and the pharmaceutically-acceptable salts thereof. The term "pharmaceutically-acceptable salts" embraces salts commonly used to form alkali metal salts and to form addition salts of free acids or free bases. Since the compounds of Formulas I-II contain basic nitrogen atoms, such salts are typically acid addition salts. The phrase "pharmaceutically-acceptable salts" is not intended to embrace quaternary ammonium salts. The nature of the salt is not critical, provided that it is pharmaceutically acceptable, and acids which may be employed to form salts are, of course, well known to those skilled in this art. Examples of acids which may be employed to form pharmaceutically acceptable acid addition salts include such inorganic acids as hydrochloric acid, sulfuric acid and phosphoric acid, and such organic acids as maleic acid, succinic acid and citric acid. Other pharmaceutically acceptable salts include salts with alkali metals or alkaline earth metals, such as sodium, potassium, calcium and magnesium, or with organic bases, such as dicyclohexylamine. All of these salts may be prepared by conventional means by reacting, for example, the appropriate acid or base with the corresponding compound of Formulas I.

## General Synthetic Procedures

Compounds of Formula I may be prepared in accordance with the following generic procedures:

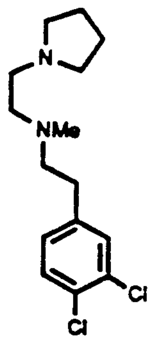

**3**

Compound **3** was synthesized in three steps starting from the readily available N-(2-chloroethyl)-pyrrolidine hydrochloride (**24**.HCl) (Scheme 1 and Table 1): methylaminolysis of **24** at ambient temperature in water in the presence of a 10-fold excess of methylamine afforded N-methyl-2-(1-pyrrolidinyl)ethylamine (**25**) in 69% redistilled yield. Coupling of this compound with 3,4-dichlorophenylacetic acid (**26**) in the presence of DCC afforded amide **27** in 98% yield. Alane ($AlH_3$) reduction[21] of **27** afforded **3** in 74% yield; as expected, use of $LiAlH_4$ to achieve this step resulted in loss of the chlorine atoms as we have observed previously for an analogous reduction.[19] Use of borane-THF complex resulted in a very stable $BH_3$-amine complex which made isolation of the product very difficult. The NH (**28**), N-ethyl (**29**), N-(n-propyl) (**30**), N-allyl (**31**) and N-(n-butyl) (**32**) intermediates (Scheme 1 and Table 1) were similarly obtained in 47-83% yields by treatment of N-(2-chloroethyl)pyrrolidine hydrochloride (24) with an excess of the corresponding primary amines under aqueous (compounds **28-30, 32**) or methanolic (compound **31**) conditions. The sequence of coupling of **28-32** with 3,4-dichlorophenylacetic (**26**) (73-100% yields) followed by alane reduction of the resultant amides (**33-37**) in THF gave target compounds **4-8**, respectively (Scheme 1 and Table 1) in 65-81% yields.

Coupling of diamine **25** with phenylacetic, 3-chlorophenylacetic, 4-chlorophenylacetic or $\beta$-naphthylacetic acids (88-100% yields) followed by $AlH_3$ reduction in THF (for amides **38-40**) or $LiAlH_4$ reduction (for naphthylacetamide **41**) yielded the aryl analogs **20-23** (62-73% yield).

Truncated compounds **9** and **10** were obtained starting with N-(2-chloroethyl)dimethylamine hydrochloride (**42**) and commercially available N,N-dimethylethylenediamine (**43**) (Scheme 2 and Table 1). Aminolysis of **42** with $MeNH_2$ afforded diamine **44** in 97% redistilled yield. The sequence of DCC mediated coupling of **43** and **44** with 3,4-dichlorophenylacetic acid (82 and 62%, respectively) followed by $AlH_3$ reduction (73 and 38%, respectively) furnished **9** and **10**. Truncated analog **11** was similarly obtained starting with N-(2-chloroethyl)diethylamine hydrochloride (**45**) (Scheme 2 and Table 1). Compounds **12** and **13** were synthesized starting with symmetrical diamines **48** and **49**, respectively (Scheme 2 and Table 1). Coupling of large excesses of **48** and **49** with 3,4-dichlorophenylacetic acid (**26**) yielded monoamides **50** (78%) and **51** - (56%) as the major products. Any neutral diamide bi-products that formed were removed extractively by acid/base partitioning. $AlH_3$-reduction of **50** and **51** in THF yielded the desired **12** (59%) and **13** (44%).

1R-(+)-and 1S-(-)- methyl analogs of parent compound **3** were synthesized starting with R-(-)-and S-(+)-N-*t*-boc protected alanines [(-)-and (+)-**52**] (Scheme 3 and Table 1). Coupling of these precursors with pyrrolidine in the presence of 1-(3-dimethylaminopropyl)-3-ethylcarbodiimide hydrochloride and hydroxybenzotriazole (HOBT) gave amides (-)-and (+)-**53**.[22, 23] $LiAlH_4$ reduction of (-)- and (+)-**53** afforded diamines S-(-)- and R-(+)-**54** (85% yield)[23]. The optical purity (>98%) of these diamines was determined by $^1H$-NMR analysis of the ureas **73** and **74** (Scheme 6) formed in quantitative yield of these amines with optically pure S-(+)-$\alpha$-methylbenzylisocyanate in $CHCl_3$[24] Coupling of 1R-(+)-and 1S-(-)-**54** with 3,4-dichlorophenylacetic acid gave amides 1R-(+)-and 1S-(-)-**55**[23], respectively. The target compounds 1R-(+)-and 1S-(-)-**14** (Scheme 3 and Table 1) were furnished by $AlH_3$ reduction[21] of 1R-(+)-and 1S-(-)-**55**, respectively. The corresponding (±)-2-methyl derivative of **9**, compound (±)-**15** (Scheme 2) was synthesized (Scheme **3** and Table 1) by methylaminolysis of N,N-dimethyl-2-chloropropylamine hydrochloride (**56**); this reaction afforded an 8.2 : 1.8 mixture of isomeric N-methyl-2-(dimethylamino)propylamine [(±)-**57**] and N,N-dimethyl-2-(methylamino)propylamine [(±)-**58**]. This distribution of products forms from nucleophilic attack of $MeNH_2$ on aziridinium cation **59** (Scheme **3**). As expected, attack of $MeNH_2$ on the loss hindered carbon atom of **59** affords the major product (±)-**57**. The major product (±)-**57** was readily separated from its isomer (±)-**58** by conversion to its *bis*oxalate salt in MeOH. DCC-mediated condensation of (±)-**57** with **3,4-**

dichlorophenylacetic acid afforded amide (±)-**60** in 73% yield. $AlH_3$ reduction of (±)-**60** gave the target compound (±)-**15** in 84% yield.

Compound **16** (Scheme **4** and Table **1**) which serves to examine the effect of increasing the N--N distance on sigma receptor affinity was synthesized starting with commercially available 1-(3-aminopropyl)-pyrrolidine (**68**). The sequence of N-formylation of **68** with refluxing ethyl formate (97%), $LiAlH_4$ reduction to the N-methyl intermediate **70** (81%), DCC-mediated coupling with 3,4-dichlorophenylacetic acid (94%) and $AlH_3$ reduction (85%) afforded the target compound, **16**. Compound **17** was synthesized by DCC-mediated coupling of 25 with 3,4-dichlorobenzoic acid (88%) followed by $AlH_3$ reduction[21] (69%).

To observe the effect of ring size, compounds **18** and **19** (Scheme 5 and Table 1) were synthesized. Compound **18** was synthesized by the sequence of methylaminolysis of **61** to give **62** (79% yield), condensation with 3,4-dichlorophenylacetic acid (100% yield) and $AlH_3$ reduction in THF (71% yield). Compound **19** was obtained starting from homopiperidine (**64**). Coupling of **64** with N-*t*-boc glycine in the presence of 1-(3-dimethylaminopropyl)-3-ethylcarbodiimide hydrochloride[25] and HOBT gave **65** in 71% yield. Intermediate **65** was reduced to diamine **66** by treatment with $LiAlH_4$ in THF. DCC-mediated coupling of **66** with 3,4-dichlorophenylacetic acid (**26**) afforded amide **67** in 75% yield which on $AlH_3$ reduction gave **19** in 73% yield.

**Scheme 1**

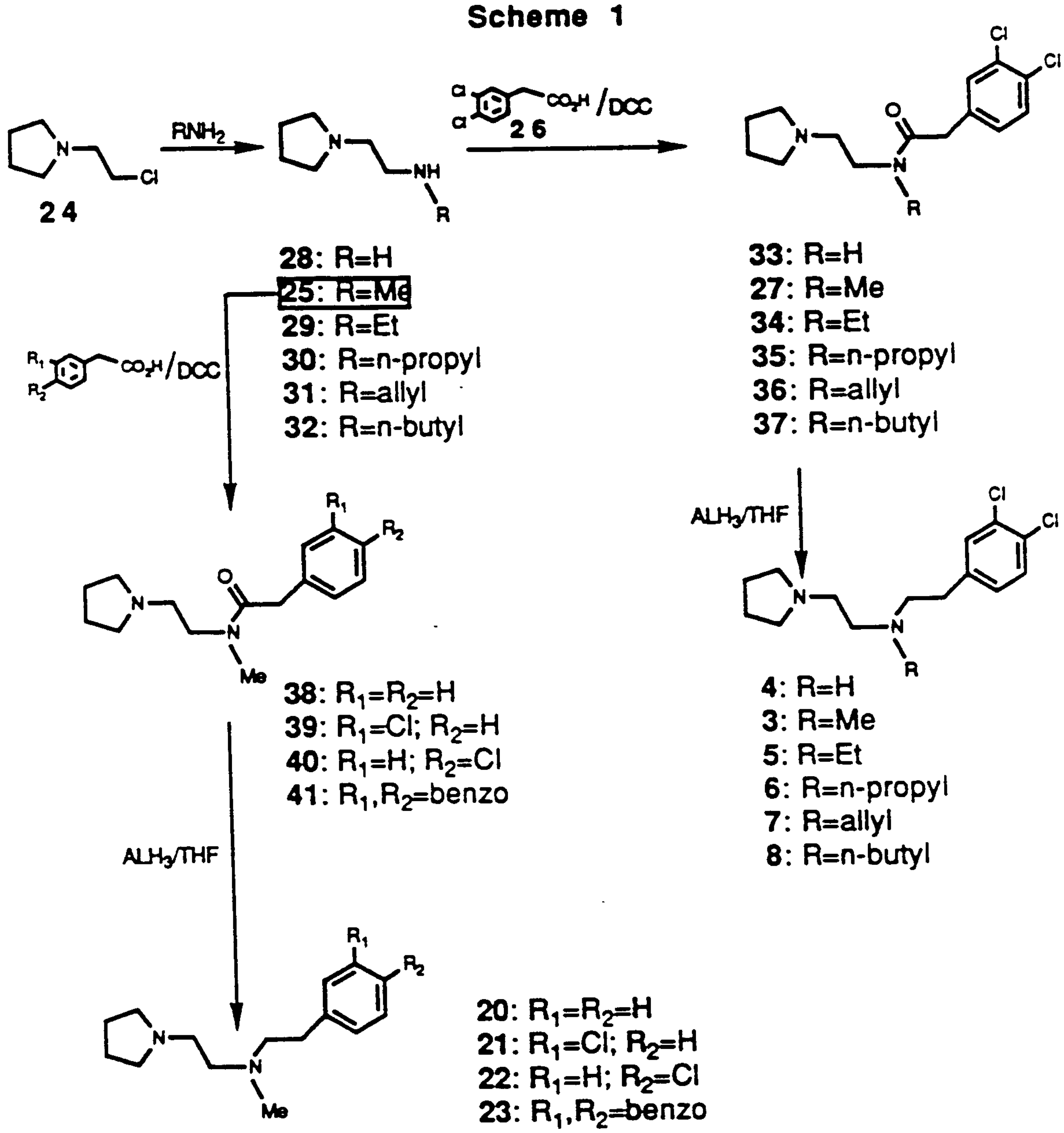

**Scheme 2**: Synthesis of Truncated Versions of N-[2-(3,4-Dichlorophenyl)ethyl]-N-methyl -2-(1-pyrrolidinyl)ethylamine

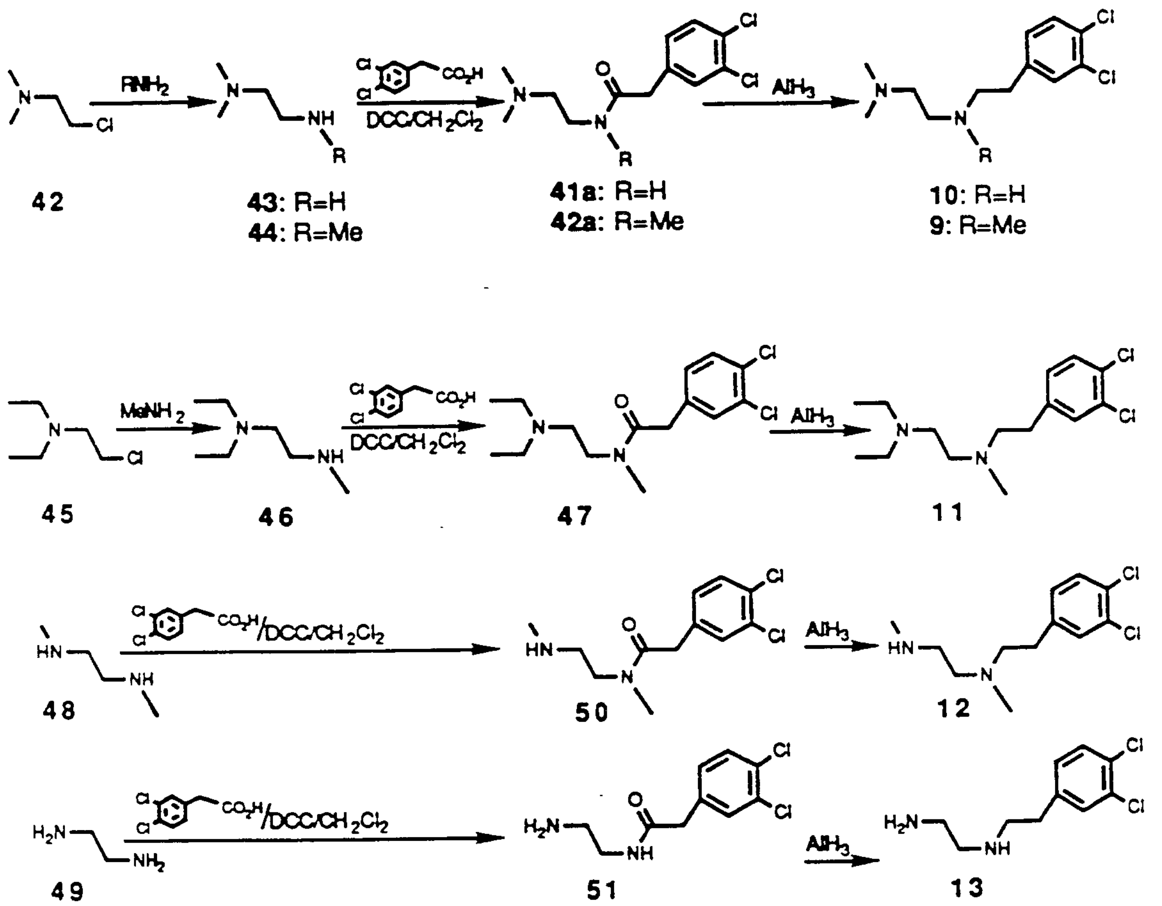


42

**43**: R=H
**44**: R=Me

**41a**: R=H
**42a**: R=Me

**10**: R=H
**9**: R=Me

45 46 47 11

48 50 12

49 51 13

**Scheme 3**: Synthesis of R-and S-N-[2-(3,4-Dichlorophenyl)ethyl]-N-methyl - 1-methyl-2-(1-pyrrolidinyl)ethylamines and (±)-N-[2-(3,4-Dichlorophenyl)ethyl]-N-methyl -2-methyl-2-(dimethylamino)ethylamine

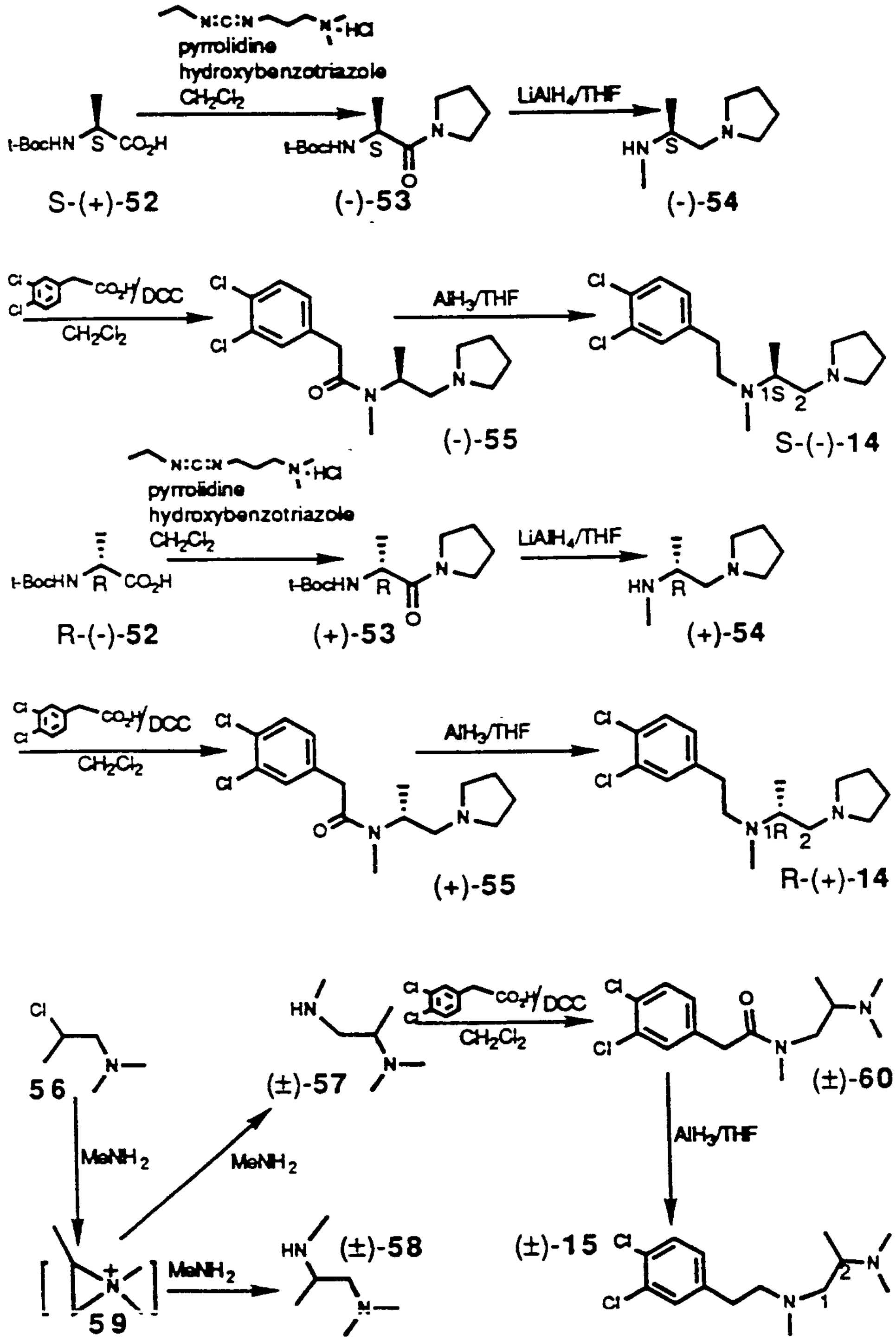


S-(+)-52

(-)-53

(-)-54

(-)-55

S-(-)-14

R-(-)-52

(+)-53

(+)-54

(+)-55

R-(+)-14

56

(±)-57

(±)-60

59

(±)-58

(±)-15

Scheme 4: Synthesis of Miscellaneous 3,4-Dichlorophenylalkyldiamines

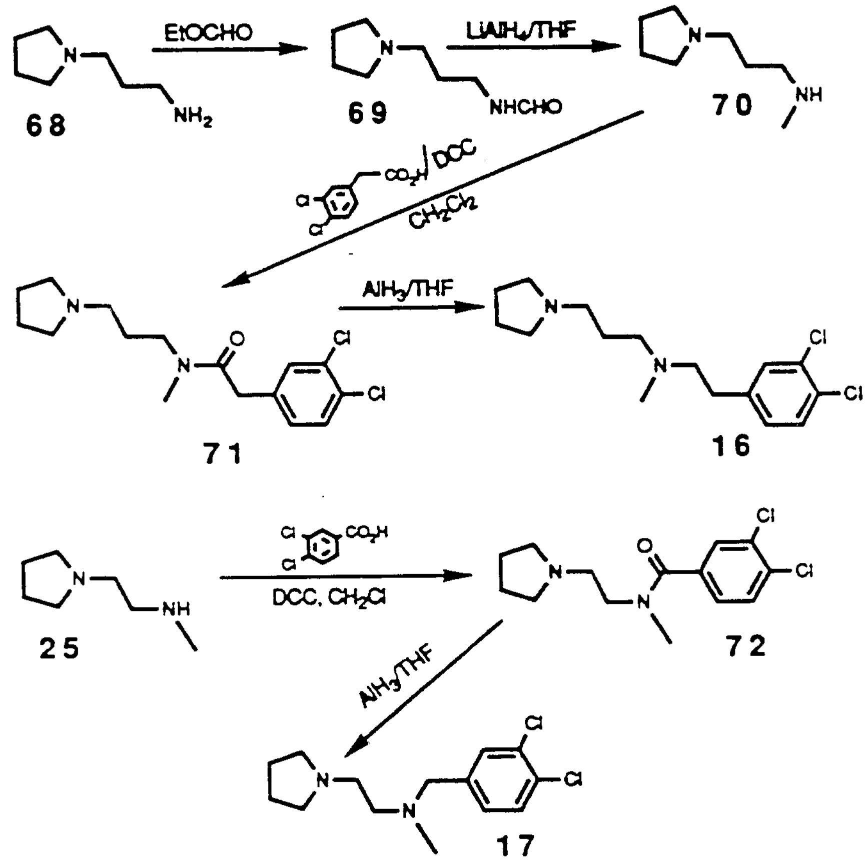


68 69 70 71 16 25 72 17

**Scheme 5**: Synthesis of Six and Seven Membered Ring Homologs of N-[2-(3,4-Dichlorophenyl)ethyl]-N-methyl -2-(1-pyrrolidinyl)ethylamine

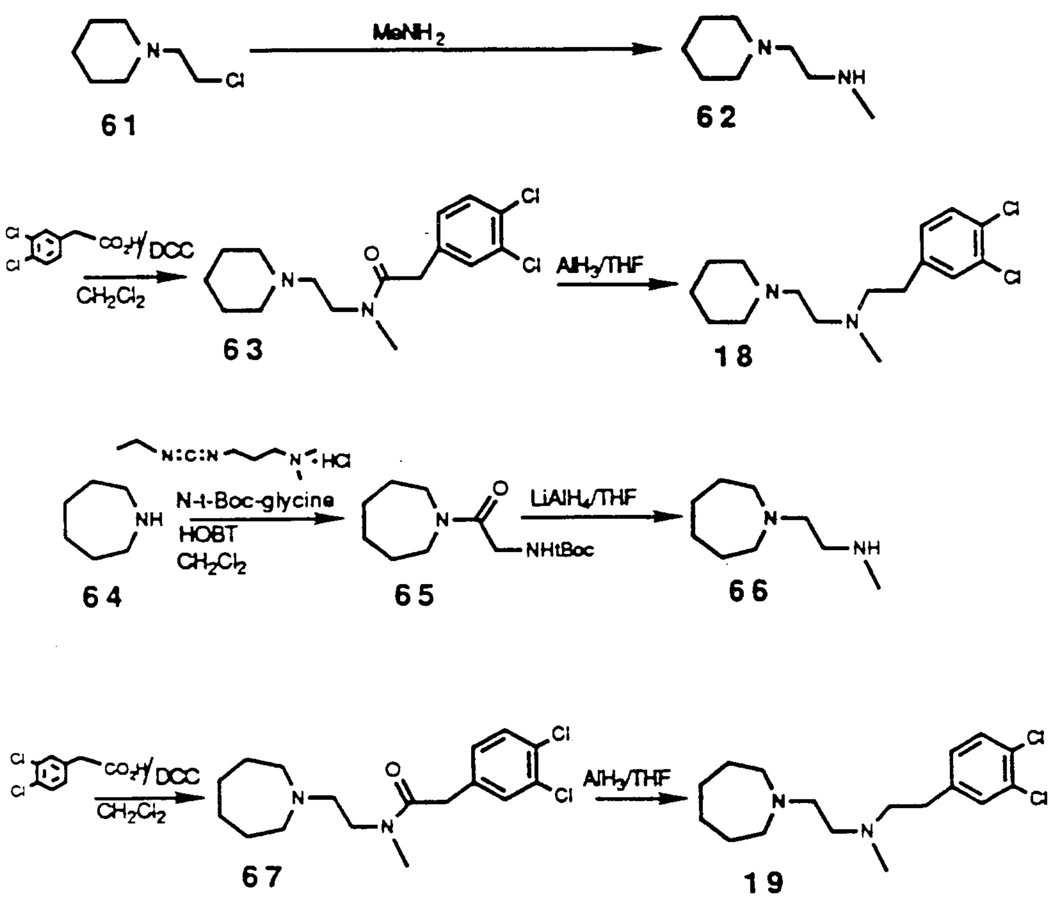


61 62 63 18 64 65 66 67 19

**Scheme 6**: Determination of Optical Purity of Chiral Diamine Intermediates (+)-and (-)-**54**

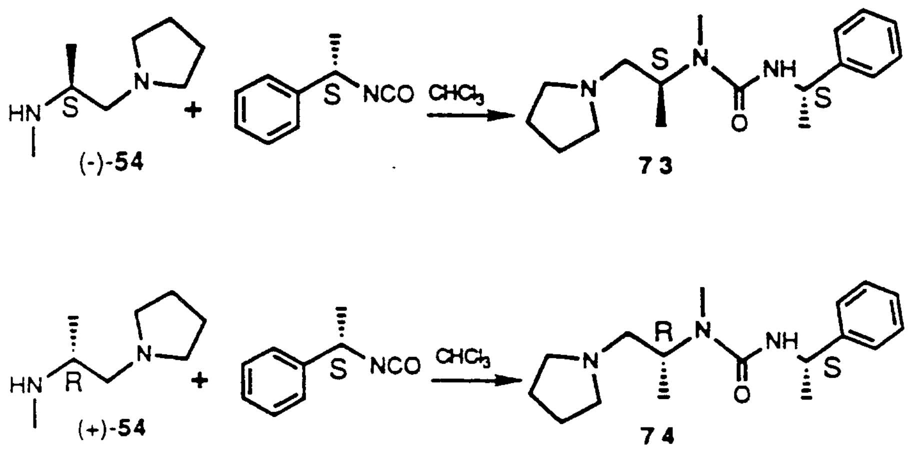


(-)-**54** 73 (+)-**54** 74

### Materials and Methods

Melting points were determined on a Thomas-Hoover capillary apparatus and are uncorrected. Specific rotation determinations at the sodium-D line were obtained in a 1 dM cell using a Perkin-Elmer 241-MC polarimeter. Elemental analyses were performed at Atlantic Microlabs, Atlanta, GA; where molecular formulae are indicated in Table 1, elemental analyses for C, H, and N are within ±0.4% of the theoretical values unless indicated otherwise. Chemical-ionization mass spectra (CIMS) were obtained using a Finnigan 1015 mass spectrometer. Electron ionization mass spectra (EIMS) and high resolution mass measurements (HRMS) were obtained using a VG-Micro Mass 7070F mass spectrometer. $^{1}$H-NMR spectra were obtained from $CDCl_3$ solutions using a Varian XL-300 spectrometer. Thin layer chromatography (TLC) was performed on 250 $\mu$M Analtech GHLF silica gel plates. TLC system A corresponds to $CHCl_3$-MeOH-conc. aq. $NH_3$ - (90:9:1) TLC system B corresponds to $CHCl_3$-MeOH-conc. aq. $NH_3$ (80:18:2). No attempt was made to optimize the yields reported in Table 1.

**N-Methyl-2-(1-pyrrolidinyl)ethylamine (25)**. *Method A*. To a stirred solution of 40% aqueous methylamine (913 mL, 11.8 mole, 10 eq) was added during 20 min a solution of N-(2-chloroethyl)pyrrolidine hydrochloride (**24**.HCl) (200 g, 1.18 mole) in distilled water (300 mL). The solution became warm during the addition. TLC (solvent system B) 30 min after completion of the addition indicated that the reaction was complete. The reaction mixture was basified by the addition of 370 g of NaOH pellets. No external cooling was necessary during the addition of NaOH since the vigorous evolution of gaseous methylamine removed the heat generated as heat of vaporization. The desired product separated as a yellow oil after complete addition of the NaOH. The oily reaction mixture was set aside and allowed to cool to room temperature. The cooled solution was extracted with ether (4 x 500 mL) and the combined organic layer was evaporated in vacuo to afford the product as a pale yellow oil. The oil was distilled under aspirator vacuum collecting the central fraction bp 95-105 °C, yield = 98 g (65%). Crystallization of the oxalate salt from MeOH afforded an analytically pure sample of **25**.bis oxalate (Table 1): $^{1}$H-NMR ($CDCl_3$) (base) $\delta$ 2.70 (t, J=6.0 Hz, 2H, $\underline{CH_2}NH$), 2.59 (t, J=6.0 Hz, 2H, $\underline{CH_2}CH_2NH$), 2.50 (m, 4H), 2.45 (s, 3H, $NH\underline{CH_3}$), 1.77 (m, 4H), 1.58 (br s, 1H, NH); CIMS (M+H)=129; $C_7H_{16}N_2$ +H requires: 129.

**N-[2-(3,4-Dichlorophenyl)acetyl]-N-methyl-2-(1-pyrrolidinyl)ethylamine (27).** *Method B*. To a stirred solution of N,N'-dicyclohexylcarbodiimide (DCC) (123 g, 0.596 mole) in $CH_2Cl_2$ (500 mL) was added a solution of 3,4-dichlorophenylacetic acid (91.7 9, 0.447 mole) in $CH_2Cl_2$ (500 mL) and the solution was stirred for 10 min at room temperature. To this solution was added **25** (38.0 g, 0.297 mole) and the solution was stirred for 20 min at room temperature when TLC (solvent system A) indicated that the reaction was complete. The precipitated N,N'-dicyclohexylurea was filtered off and the filter cake was washed with ether (500 mL) The filtrate was diluted to a total volume of 1500 mL with ether. The diluted filtrate was extracted with 10% aqueous citric acid (1000 mL) and the organic layer was discarded. The aqueous citric acid extract was washed with a further 2 x 500 mL of ether, basified by addition of excess concentrated aqueous ammonia solution and then extracted with 2 x 500 mL of $CH_2Cl_2$. The $CH_2Cl_2$ layer was back-washed with water (200 mL), dried over $Na_2SO_4$ and evaporated to give 91.3 g (98%) of **27** as a colorless oil which was of high enough purity (**99** % by G.C. analysis) for use in the next step. For purposes of further characterization, a small amount of this material was converted to **27**.HCl in ethyl acetate (Table 1): $^{1}$H-NMR ($CDCl_3$) (base) $\delta$ 7.35-7.40 (m, 2H), 7.11 (dd, J=2 Hz, J=8.3 Hz, 1H), 3.69 (40%), 3.65 (60%) (s, 2H, $CO\underline{CH_2}$), 3.54 (60%), 3.43 (40%) (t, J=7.1 Hz, 2H, $CH_2\underline{CH_2}N(CH_3)$), 3.03 (60%), 2.97 (40%) (s, 3H, $N\underline{CH_3}$), 2.62 (m, 2H, $\underline{CH_2}CH_2N(CH_3)$), 2.54 (m, 4H), 1.76 (m, 4H); CIMS (M+H)=315; $C_{15}H_{20}Cl_2N_2O$+H requires: 315.

**N-[2-(3,4-Dichlorophenyl)ethyl]-N-methyl-2-(1-pyrrolidinyl)ethylamine (3)**. *Method C*. To a stirred solution of freshly prepared alane (1450 mL of a 1.0M solution in THF prepared as described below and in ref **21**) (1.45 mole) was added dropwise during 15 min, a solution of **27** base (91.2 g, 0.289 mole). In order to prevent the reaction mixture from heating to the point of reflux of the THF, it was periodically immersed in an ice-bath to keep the temperature around room temperature. Five min after addition of **27** was complete, progress of the reaction was monitored by TLC (solvent system A); the TLC indicated that the reduction was complete. The reaction mixture was carefully poured into a large beaker (2-4 liter) containing 600 mL of 15% aqueous NaOH and the mixture was vigorously stirred during the addition. After the quenching was complete, the aqueous mixture was cooled to room temperature and enough chloroform (approx 1500 mL) was added to result in a lower organic layer. The organic extract was separated, dried through a pad of $Na_2SO_4$ and the solvent was evaporated to give the crude product as a pale yellow oil. The oil was dissolved in MeOH to a volume of 1000 mL and the stirred solution was heated to boiling point and then the heat was removed. To the stirred solution was added 100 mL of 47% HBr. The solution continued to boil as a result of heat of neutralization. After the addition of the HBr was complete and the

boiling had subsided, crystallization began to occur spontaneously. During the crystallization process, the solution continued to boil as a result of heat of crystallization. The flask was set aside to cool to room temperature and the crystals were filtered and washed twice with cold (0 °C) MeOH, once with ether, and dried in vacuo at 80 °C to give analytically pure **3**.HBr (99 g, 74%) (Table 1): $^1$H-NMR ($CDCl_3$) (base) δ 7.34 (d, J = 8.2 Hz, 1H, Ar-H(5)), 7.29 (d. J = 2.0 Hz, 1H, Ar-H(2)), 7.03 (dd, J = 8.2 Hz, J = 2.0 Hz, 1H, Ar-H(6)), 2.73 (m, 2H), 2.63 (m, 2H), 2.58 (br s, 4H), 2.51 (m, 4H), 2.32 (s, 3H, $NCH_3$), 1.78 (m, 4H); CIMS (M + H)-= 301; $C_{15}H_{22}Cl_2N_2$ + H requires: 301.

**N-Allyl-2-(1-pyrrolidinyl)ethylamine (31).** *Method D.* To a stirred solution of allylamine (38.7 g, 0.679 mole) in MeOH (200 mL) was added dropwise at room temperature a solution of 1-(2-chloroethyl)pyrrolidine hydrochloride (**24**.HCl) (20.0 g, 0.177 mole) in MeOH (50 mL). Analysis of the reaction by TLC (solvent system B) indicated that the reaction was complete after 4 days. The solvent was evaporated in vacuo, and the oily residue was dissolved in distilled water (200 mL), cooled to 0 °C, and treated with KOH pellets (60 g) with continued cooling from an ice bath. A separate organic layer of crude **31** formed after complete addition of the KOH. The cooled mixture was extracted with CHCl3 (2 x 100 mL) and the combined extract was evaporated to give a yellow oil; Distillation under high vacuum afforded **31** (11.6 g, 64%) as a colorless oil: bp 73°/0.8 mmHg (Table 1): $^1$H-NMR ($CDCl_3$) (base) δ 5.91 (m, 1H, $CH_2CH=CH_2$), 5.17 (d, $J_{trans}$ = 17.2 Hz, 1H, $CH=CH_2$), 5.08 (d, $J_{cis}$ = 10.3 Hz, 1H, $CH=CH_2$), 3.26 (d, J = 6.0 Hz, 2H, $CH_2$-$CH=CH_2$). 2.73 (t, J = 6.5 Hz, 2H, $CH_2CH_2NH$), 2.60 (t, J = 6.5 Hz, 2H, $CH_2CH_2NH$), 2.50 (m, 4H), 1.76 (m, 4H), 1.65 (br s, 1H, NH); CIMS (M + H) = 155; $C_9H_{18}N_2$ + H requires: 155.

**N,N,N'-trimethylethylenediamine (44).** The same procedure was used as for the synthesis of **25** starting with N-(2-chloroethyl)dimethylamine hydrochloride (**42**.HCl, Scheme 2) (50 g, 0.347 mole) in 100 mL of water and aqueous methylamine (40%) (404 mL, 15 eq). Reaction was complete within 1h (TLC; solvent system A). The product was isolated by addition of NaOH (150 g) and extraction with $CHCl_3$ (3 x 200 mL). Yield = 34.3 g (97%) as a colorless oil (Table 1): $^1$H-NMR ($CDCl_3$) (base) δ 2.65 (t, J = 6.0 Hz, 2H, $CH_2CH_2NH$), 2.44 (s, 3H, $NHCH_3$), 2.40 (t, J = 6.0 Hz, 2H, $CH_2CH_2NH$), 2.22 (s, 6H, $N(CH_3)_2$), 1.61 (br s, 1H, NH); CIMS (M + H) = 103; $C_5H_{14}N_2$ + H requires: 103.

**N-(3-Formamidopropyl)pyrrolidine (69).** *Method E*. A mixture of n-(3-aminopropyl)pyrrolidine (Fluka) (**68**) (20.3 g, 158.6 mmol) and ethyl formate (150 mL) was refluxed overnight under an argon atmosphere when TLC (solvent system A) indicated that the reaction was complete. The solvent was evaporated in vacuo and the oily residue was distilled in vacuo to give **69** (24.0 g, 97%) as a colorless oil which formed a crystalline oxalate salt (Table 1): $^1$H-NMR ($CDCl_3$) (base) δ 8.13 (s, 1H, NHCHO), 7.28 (br s, 1H, NHCHO), 3.39 (q, J = 5.9 Hz, 2H, $CH_2NHCHO$), 2.59 (t, J = 6.5 Hz, 2H, $CH_2CH_2$-(1-pyrrolidine)), 2.53 (m, 4H), 1.78 (m, 4H), 1.73 (m, 5H, N-$CH_2CH_2CH_2NH$); CIMS (M + H) = 157; $C_8H_{16}N_2O$ + H requires: 157.

(±)-**2-(Dimethylamino)-N-methylpropylamine [**(±)-**57]**. To a stirred solution of aqueous methylamine (40%) (246 mL, 10 eq) at room temperature was added dropwise, a solution of 2-chloro-N,N-dimethypropylamine hydrochloride (**56.**HCl) (50 g, 316.5 mmol) in water (100 mL). Reaction was found to be complete within 30 min (TLC; solvent system A). KOH pellets (103.8 g were added and the product was isolated by extraction with $CHCl_3$ (3 x 200 mL) as described earlier for **25** to give a mixture of (±)-**57** together with (±)-**58** as the minor isomer (8.2:1.8 ratio as determined by $^1$H-NMR comparison of the C(Me) group of (±)-**57** with that of (±)-**58**): yield 32.9 g as a colorless liquid which was subjected to separation as described below.

**Separation of** (±)-**57** and (±)-**58**: Mixed amines **[**(±)-**57** and (±)-**58**) from above (25.4 g, 0.219 mol) was dissolved in MeOH and the solution was heated to boiling point and the heat source removed. To this stirred solution was added a boiling solution of oxalic acid (39.4 g, 0.44 mol) in MeOH (200 mL). The solution began to boil again during the addition as a result of heat of neutralization. As soon as all of the oxalic acid had been added, exothermic crystallization occurred. The crystallization mix was cooled to room temperature and filtered to give (±)-**57**.oxalate (Table 1): $^1$H-NMR ($CDCl_3$) (base) δ 2.74 (m, 1H, CH), 2.54 (dd, J = 4.6 Hz, 11.8 Hz, 1H, $CH_2$), 2.43 (dd, J = 5.2 Hz, 11.8 Hz, 1H, $CH_2$), 2.43 (s, 3H, $NHCH_3$), 2.21 (s, 6H, $N(CH_3)_2$), 1.81 (br s, 1H, NH), 0.904 (d, J = 6.6, Hz, 3H, $CH(CH_3)$); CIMS (M + H) = 117; $C_6H_{16}N_2$ + H requires: 117.

**N-[3-(Methylamino)propyl]pyrrolidine (70).** *Method F*. To a stirred suspension of $LiAlH_4$ (7.55 g, 199 mmol) in dry THF (200 mL) was added dropwise with cooling from an ice bath, a solution of **69** (7.55 g, 48.4 mmol) in THF (200 mL). The solution was stirred at room temperature under an $N_2$ atmosphere overnight, or until complete by TLC (solvent system B). The reaction was quenched by dropwise (with cooling) addition of water (7.55 mL), 15% NaOH (7.55 mL) and finally water (22.6 mL). The precipitated aluminum salts were filtered and the filter-cake was washed with THF (100 mL). The combined filtrate and washings were evaporated in vacuo to give **70** in quantitative yield as a colorless oil: bp 64 °C/1.3 mmHg; **70**.fumarate (Table 1): $^1$H-NMR ($CDCl_3$) (base) δ 2.63 (t, J = 7.0 Hz, 2H, $CH_2CH_2NH$), 2.49 (t, J = 7.1 Hz, 2H,

$CH_2CH_2$-(1-pyrrolidine), 2.49 (m, 4H), 2.43 (s, 3H, $NHCH_3$), 1.77 (m, 4H), 1.72 (m, 2H, $CH_2CH_2CH_2$); CIMS (M + H) = 143; $C_8H_{18}N_2$ + H requires: 143.

**N-[2-(N'-methylamino)ethyl]-N-methyl-3,4 dichlorophenylacetamide (50).** *Method G*. The complex formed by allowing 3,4-dichlorophenylacetic acid (4.1 g, 20.0 mmol) and N,N'-dicyclohexylcarbodiimide (5.5 g, 26.7 mmol) to react together in $CH_2Cl_2$ (50 mL) was added in one portion to a rapidly stirred solution of N,N'-dimethylethylenediamine (Aldrich) (17.62 g, 10 eq) in $CH_2Cl_2$ (50 mL). After stirring overnight, the solution was filtered and the filter-cake was washed with ether. The combined filtrate and washings were diluted to 200 mL with ether and extracted with 10% aqueous citric acid (200 mL). The aqueous layer was washed with ether (2 x 100 mL), basified by addition of excess concentrated aqueous $NH_3$ and extracted with $CH_2Cl_2$ (5 x 50 mL). The combined $CH_2Cl_2$ extract was dried ($Na_2SO_4$) and the solvent was evaporated *in vacuo* to give **50** (4.3 g, 78%) as a colorless oil: **50**.oxalate crystallized from EtOH (see Table 1); $^1$H-NMR ($CDCl_3$) (base) δ 7.38 (d, J = 8.2 Hz, 1H, Ar-H(5)), 7.37 (d, J = 1.7 Hz, 1H, Ar-H(2)), 7.11 (dd, J = 1.7 Hz, 8.2 Hz, 1H, Ar-H(6)), 3.72 (36%), 3.66 (64%) (s, 2H, $COCH_2$), 3.52 (64%), 3.42 (36%) (t, J = 6.5 Hz, 2H, $CH_2NCH_3$), 3.04 (64%), 2.97 (36%) (s, 3H, $CONCH_3$), 2.76 (t, J = 6.5 Hz, 2H, $CH_3NHCH_2CH_2N$-($CH_3$)CO), 2.45(40%), 2.44(60%) (s, 3H, $CH_3NH$), 1.55 (br s, 1H, NH); CIMS (M + H) = 275; $C_{12}H_{16}Cl_2N_2O$ + H requires: 275.

**Preparation of 1.0 M Alane in THF[21].** To a vigorously stirred solution of $LiAlH_4$ in THF (1600 mL of a 1.0 M solution) was added , dropwise from a dropping funnel, sulfuric acid (78.08 g, 0.5 eq) at such a rate that the solution temperature was maintained at 20 °C; this was accomplished by periodic dipping of the the reaction vessel in an ice-bath. When the addition was complete, the solution was stirred for a further 1h at 20 °C and then allowed to stand overnight at this temperature until the precipitated $Li_2SO_4$ had settled to the bottom of the flask. The 1.0 M alane was decanted from these salts prior to use. The shelf-life (2 weeks) could be increased to 1 year by decanting the 1.0 M alane solution into a clean dry bottle and storing it under a nitrogen atmosphere at -30 °C.

**N-[2-(Phenyl)ethyl]-N-methyl-2-(1-pyrrolidinyl)ethylamine (20).** *Method H.* To a stirred solution of $LiAlH_4$ in THF (72.4 mL of a 1.0 M solution, 72.4 mmol) was added dropwise from a dropping funnel, a solution of amide **38** (8.9 g, 36.2 mmol) in dry THF (50 mL). TLC (solvent system A) indicated the reaction to be almost complete after **10** min at room temperature. The reaction was driven to completion by brief refluxing and then cooled to room temperature. Additional THF (200 mL) was added and then excess $LiAlH_4$ was destroyed by dropwise addition of water (2.75 mL), 15% aqueous NaOH (2.75 mL) and finally water (8.25 mL). The reaction mixture was stirred for 1h, filtered , and the filtrate was evaporated to give the crude **20** as an oil which formed crystalline **20**.HCl (8.06 g) from EtOH (50 mL) on addition of concentrated HCl (6 mL) (Table 1): $^1$H-NMR ($CDCl_3$) (base) δ 7.30 (m, 2H), 7.20 (m, 3H), 2.78 (m, 2H), 2.65 (m, 2H), 2.61 (br s, 4H), 2.52 (m, 4H), 2.34 (s, 3H, $NCH_3$), 1.77 (m, 4H); CIMS (M + H) = 233; $C_{15}H_{24}N_2$ + H requires: 233.

**R-( + )-N-(Tertbutyloxycarbonyl)-N',N'-(tetramethylene)alaninamide [R-( + )-53].** *Method I.* A solution of *t*-Boc-D-alanine (13.2 g, 0.0696 mol), HOBT (7.5 g, 0.0556 mol) and 1-(3-dimethylaminopropyl)-3-ethylcarbodiimide hydrochloride (20 g, 0.1043 mol) in 30 mL of $CH_2Cl_2$ was stirred at room temperature for one hour. Pyrrolidine (5.9 g, 0.0835 mol) was then added, and the mixture was stirred for additional three hours. The solvent was removed in vacuo, and the mixture was diluted in 50 mL of EtOAc. This organic layer was washed with water (2x50 mL), 5% citric acid (2x50 mL), saturated $NaHCO_3$ (2x50 mL) and dried with $Na_2SO_4$. A colorless oil (10.68 g, 100%) was obtained, which was used in the next step without further purification. For the purposes of characterization, however, this compound was obtained in analytically pure form by recrystallization from isooctane (Table 1): $R_f$ = 0.24 (hexane:EtOAc = 1:1); $^1$H-NMR ($CDCl_3$) δ 5.45 (bd, 1H, J = 7.5 Hz), 4.41 (m, 1H), 3.34-3.62 (m, 4H), 1.79-1.99 (m, 4H), 1.41 (bs, 9H), 1.28 (d, 3H, J = 6.8 Hz); CIMS (M + H) = 243; $C_{12}H_{22}N_2O_3$ + H requires: 243.

**R-( + )-2-(1-Pyrrolidinyl)-1-methyl-N-methylethylamine [R-( + )-54].** *Method J.* R-()-N-(*tert*Butyloxycarbonyl)-N,N'-(tetramethylene)alaninamide (8.4 g, 0.0348 mol) was dissolved in 100 mL of THF. A 1M solution of $LiAlH_4$ in THF was then added dropwise (100 mL, 0.1044 mol) and the mixture was refluxed for three hours. Water (4 mL) was added, then 10% NaOH (4 mL) and water again (8 mL). Filtration led to a clean solution which was diluted with $Et_2O$ (100 mL). This organic layer was extracted with 5% citric acid (3x60 mL). The aqueous solution was basified with KOH pellets and extracted with $CHCl_3$ (4x40 mL). The resulting organic layer was dried ($Na_2SO_4$) and the solvent was removed in vacuo to give 4.94 g (100%) of crude diamine. This could be purified by crystallization of its oxalate salt from EtOH to give **54**.bisoxalate (85% based on amount of pyrrolidine) (see Table 1): $^1$H-NMR ($CDCl_3$) δ 2.54 (complex m, 4H, pyrrolidinyl 2-H, 5-H), 2.42 (s, 3H, NMe), 2.40 (complex m, 2H), 2.05 (dd, 1H, J = 11 Hz, J = 2.8 Hz, $CH_2$-N), 2.02 (br s, 1H, NH), 1.75 (complex m, 4H, pyrrolidinyl 3-H, 4-H), 0.99 (d, J = 5.9 Hz, CHMe); CIMS (M + H)- = 143; $C_8H_{18}N_2$ + H requires: 143.

**N-[1-(1-Pyrrolidinyl)-2-*S*-propyl]-N-methyl-N'-[*S*-α-methylbenzyl]urea (73).** *Method K.* To a stirred

solution of S-(-)-**54** (12.1 mg, 0.0854 mmol) in dry $CHCl_3$ (0.5 mL) was added S-(-)-1-phenylethylisocyanate (12.6 mg, 0.0854 mmol) and the solution was stirred at room temperature under an N2 atmosphere until complete by TLC (solvent system A). The solvent was evaporated *in vacuo* to give **73** in quantitative yields as a colorless oil (Table 1): $^1$H-NMR ($CDCl_3$) $\delta$ 7.32 (m, 5H), 6.13 (br s, 1H), 4.95 (m, 1H), 4.24 (br s, 1H), 2.73 (s, 3H), 2.42-2.65 (m, 5H), 2.32 (dd, 1H, J = 12.8 Hz, 4.1 Hz), 1.55 (m, 4H), 1.46 (d, 3H, J = 6.8 Hz,), 1.11 (d, 3H, J = 7.0 Hz); HRMS $M^+$ (found): 289.2145; $M^+$($C_{17}H_{27}N_3O$) requires: 289.2154.

**N-[1-(1-Pyrrolidinyl)-2-*R*-propyl]-N-methyl-N'-[*S*-α-methylbenzyl]urea (74).** The same method was followed as described above for **73** starting with R-(+)-**54** (16.5 mg, 0.116 mmol), dry $CHCl_3$ (0.5 mL), S-(-)-1-phenylethylisocyanate (12.6 mg, 0.0854 mmol) to give **74** in quantitative yield as a colorless oil (Table 1): $^1$H-NMR ($CDCl_3$) $\delta$ 7.33 (m, 5H), 6.22 (br s, 1H), 4.98 (m, 1H), 4.27 (br s, 1H), 2.73 (s, 3H), 2.50-2.68 (m, 5H), 2.35 (dd, 1H, J = 12.9, 4.0 Hz), 1.71 (m, 4H), 1.45 (d, 3H, J = 6.9 Hz), 1.11 (d, 3H, J = 7.0 Hz); HRMS M + (found): 289.2144; M + ($C_{17}H_{27}N_3O$) requires: 289.2154.

**Table 1: Physical and chemical properties of target compounds and their intermediates**

| Compound | Molecular Formula | Solvent[a] | Method | % Yield[b] | Mp (°C) |
|---|---|---|---|---|---|
| 3.HBr[c] | $C_{15}H_{24}Br_2Cl_2N_2$ | EtOH | C | 74 | 264-265 (dec) |
| 4.HBr | $C_{14}H_{22}Br_2Cl_2N_2$ | EtOH | C | 70 | 244-245 (dec) |
| 5.HBr | $C_{16}H_{26}Br_2Cl_2N_2$ | EtOH | C | 77 | 188-189 |
| 6.HBr | $C_{17}H_{28}Br_2Cl_2N_2$ | EtOH | C | 65 | 190-191 |
| 7.HBr | $C_{17}H_{26}Br_2Cl_2N_2$ | 2-PrOH | C | 78 | 178-179 |
| 8.oxalate | $C_{22}H_{32}Cl_2N_2O_8$ | MeOH | C | 81 | 203-204 (dec) |
| 9.HBr | $C_{13}H_{22}Br_2Cl_2N_2$ | EtOH | C | 38 | 253-255 (dec) |
| 10.HBr | $C_{12}H_{20}Br_2Cl_2N_2$ | EtOH | C | 73 | 222-223 (dec) |
| 11.HBr | $C_{15}H_{26}Br_2Cl_2N_2$ | EtOH | C | 68 | 179-180 |
| 12.HBr | $C_{12}H_{20}Br_2Cl_2N_2$ | EtOH | C | 59 | 215-217 |
| 13.HBr | $C_{10}H_{16}Br_2Cl_2N_2$ | EtOH | C | 44 | 264-267 (dec) |
| (R)-14. oxalate[d] | $C_{20}H_{28}Cl_2N_2O_8$ | MeOH/DMF (2:1) | C | 52 | 198-200 |
| (S)-14. oxalate[e] | $C_{20}H_{28}Cl_2N_2O_8$ | MeOH/DMF(2:1) | C | 50 | 198-200 |
| (±)-15. oxalate | $C_{18}H_{26}Cl_2N_2O_8$ | EtOH | C | 84 | 152-153 |
| 16.HBr | $C_{16}H_{26}Br_2Cl_2N_2$ | MeOH | C | 85 | 269-270 (dec) |
| 17.HCl[c] | $C_{14}H_{22}Cl_4N_2$ | EtOH | C | 69 | 258-259 (dec) |
| 18.HBr | $C_{16}H_{26}Br_2Cl_2N_2$ | MeOH | C | 71 | 264-265 (dec) |
| 19.HCl | $C_{17}H_{28}Cl_4N_2$ | MeOH | C | 73 | 260.5-261.5 (dec) |
| 20.HCl | $C_{15}H_{26}Cl_2N_2$ | EtOH | H | 73 | 266-267 (dec) |
| 21.HCl | $C_{15}H_{25}Cl_3N_2$ | EtOH | C | 62 | 258-259 (dec) |
| 22.HCl | $C_{15}H_{25}Cl_3N_2$ | EtOH | C | 67 | 257-258 (dec) |

**Table 1 Continued**: Physical and chemical properties of target compounds and their intermediates

| Compound | Molecular Formula | Solvent[a] | Method | % Yield[b] | Mp (°C) |
|---|---|---|---|---|---|
| **23**.HBr | $C_{19}H_{28}Br_2N_2$ | DMF | H | 71 | 259-260 (dec) |
| **25**.oxalate | $C_{11}H_{20}N_2O_8$ | MeOH | A | 69 | 206-207[f] |
| **27**.HCl | $C_{15}H_{21}Cl_3N_2O$ | EtOAc | B | 98 | 169.5-170.5[g] |
| **28**.fumarate | $C_{14}H_{22}N_2O_8$ | MeOH | A | 47 | 165-166 |
| **29**.oxalate | $C_{12}H_{22}N_2O_8$ | MeOH | A | 54 | 230-231 (dec)[h] |
| **30**.HCl | $C_9H_{22}Cl_2N_2$ | EtOH | A | 83 | 211-212[i] |
| **31**.fumarate | $C_{17}H_{26}N_2O_8$ | EtOH | D[j] | 64 | 133-135[k] |
| **32**.fumarate | $C_{18}H_{30}N_2O_8$ | EtOH | A[j] | 80 | 163-164[l] |
| **33** (base) | $C_{14}H_{18}Cl_2N_2O$ | isooctane/EtOAc | B | 73 | 74-75 |
| **34**.oxalate | $C_{18}H_{24}Cl_2N_2O_5$ | 2-PrOH | B | 91 | 143.5-144 |
| **35**.oxalate | $C_{19}H_{26}Cl_2N_2O_5$ | 2-PrOH | B | 96 | 170.5-171.5 |
| **36**.fumarate | $C_{21}H_{26}Cl_2N_2O_5$ | EtOH | B | 97 | 153-154 |
| **37**.oxalate | $C_{20}H_{28}Cl_2N_2O_5$ | 2-PrOH | B | 100 | 150.5-151 |
| **38**.oxalate | $C_{17}H_{24}N_2O_5$ | 2-PrOH | B | 100 | 159-160 |
| **39**.oxalate | $C_{17}H_{23}ClN_2O_5$ | 2-PrOH | B | 100 | 142.5-143 |
| **40**.oxalate | $C_{17}H_{23}ClN_2O_5$ | 2-PrOH | B | 88 | 151-153 |
| **41**.fumarate | $C_{23}H_{28}N_2O_5$ | 2-PrOH | B | 100 | 128-130 |
| **41a** (base) | $C_{12}H_{16}Cl_2N_2O$ | isooctane | B | 82 | 63-64 |
| **42a**.HCl | $C_{13}H_{19}Cl_3N_2O$ | 2-PrOH | B | 62 | 168-169 |
| **44** fumarate | $C_{13}H_{22}N_2O_8$ | MeOH | A | 97 | 176-176.5 |
| **46**.fumarate | $C_{15}H_{26}N_2O_8$ | 2-PrOH | A | 77 | 116-117 |
| **47**.oxalate | $C_{17}H_{24}Cl_2N_2O_5$ | EtOAc | B | 76 | 100-101 |
| **50**.oxalate | $C_{14}H_{18}Cl_2N_2O_5$ | EtOH | G | 78 | 193-195 |
| **51**.fumarate | $C_{14}H_{16}Cl_2N_2O_5$ | 2-PrOH | G | 56 | 133-135 |
| **(S)-(-)-53**[m,n] | $C_{12}H_{22}N_2O_3$ | isooctane | I | 100 | 69-70 |

**Table 1 Continued**: Physical and chemical properties of target compounds and their intermediates

| Compound | Molecular Formula | Solvent[a] | Method | % Yield[b] | Mp (°C) |
|---|---|---|---|---|---|
| (R)-(+)-53[o,p] | $C_{12}H_{22}N_2O_3$ | isooctane | I | 100 | 65.5-67 |
| (S)-(-)-54 .oxalate[q] | $C_{12}H_{22}N_2O_8$ | EtOH | J | 83 | 141-144 |
| (R)-(+)-54 .oxalate[r] | $C_{12}H_{22}N_2O_8$ | EtOH | J | 85 | 141-144 |
| (S)-(-)-55 .oxalate[s] | $C_{18}H_{24}Cl_2N_2O_5$ | 2-PrOH | B | 70 | 114-115[t] |
| (R)-(+)-55 .oxalate[u] | $C_{18}H_{24}Cl_2N_2O_5.H_2O$ | 2-PrOH | B | 72 | 114-115[v] |
| (±)-57.oxalate | $C_{10}H_{20}N_2O_8$ | MeOH | A | 90[w] | 170-172 |
| (±)-60.oxalate | $C_{16}H_{22}Cl_2N_2O_5$ | 2-PrOH | B | 73 | 151.5-152.5 |
| 62.fumarate | $C_{16}H_{26}N_2O_8$ | EtOH | A | 79 | 161-162 |
| 63.oxalate | $C_{18}H_{24}Cl_2N_2O_5$ | 2-PrOH | B | 100 | 148-149 |
| 65[x] | $C_{13}H_{24}N_2O_3$ | ------ | I | 71 | oil |
| 66.oxalate | $C_{13}H_{24}N_2O_8$ | MeOH | J | 80 | 170-180 |
| 67.oxalate | $C_{19}H_{26}Cl_2N_2O_5$ | 2-PrOH | B | 75 | 142-143 |
| 69.oxalate | $C_{10}H_{18}N_2O_5$ | 2-PrOH | E | 97 | 114-116 |
| 70.fumarate | $C_{16}H_{26}N_2O_8$ | 2-PrOH | F | 81 | 140-141[y] |
| 71.fumarate | $C_{20}H_{26}Cl_2N_2O_5$ | 2-PrOH | B | 94 | 147-147.5 |
| 72.fumarate | $C_{18}H_{22}Cl_2N_2O_5$ | 2-PrOH | B | 88 | 149-150 |
| 73 (base) | $C_{17}H_{27}N_3O$ | ----- | K | 100 | oil[z] |
| 74 (base) | $C_{17}H_{27}N_3O$ | ----- | K | 100 | oil[aa] |

Table 2: Sigma receptor binding affinities of N-alkyl substituted N-[2-(3,4-dichlorophenyl)ethyl]-2-(1-pyrrolidinyl)ethylamines

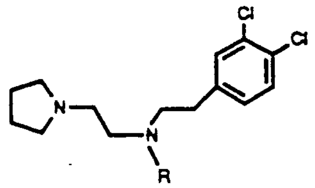

| Compound | Code | R | $K_i$ (nM) ([$^3$H]-(+)-3-PPP) |
|---|---|---|---|
| 4 | BD1060 | H | 1.11±0.11 |
| 3 | BD1008 | Me | 0.34±0.07 |
| 5 | BD1067 | Et | 0.51±0.08 |
| 6 | BD1121 | n-Pr | 1.35±0.15 |
| 7 | BD1052 | allyl | 1.39±0.03 |
| 8 | BD1069 | n-Bu | 11.7±2.5 |

Table 3: Sigma receptor affinities of truncated forms of N-[2-(3,4-dichlorophenyl)ethyl]-N-methyl-2-(1-pyrrolidinyl)ethylamine (3)

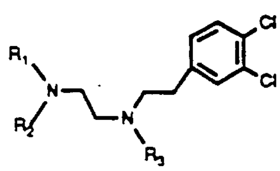

| Compound | Code | $R_1$ | $R_2$ | $R_3$ | $K_i$ (nM) ([$^3$H]-(+)-3-PPP) |
|---|---|---|---|---|---|
| 3 | BD1008 | $-(CH_2)_4-$ | | Me | 0.34±0.07 |
| 9 | BD1047 | Me | Me | Me | 1.26±0.26 |
| 10 | BD1061 | Me | Me | H | 20.18±0.22 |
| 11 | BD1120 | Et | Et | Me | 0.73±0.11 |
| 12 | BD1139 | Me | H | Me | 5.78±0.45 |
| 13 | BD1142 | H | H | H | 277.0±75.6 |

Table 4: Sigma receptor affinities of 1- and 2-methyl analogs of 3 and 9

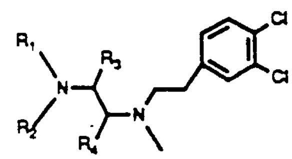

| Compound | Code | $R_1,R_2$ | $R_3$ | $R_4$ | $K_i$ (nM) ([$^3$H]-(+)-3-PPP) |
|---|---|---|---|---|---|
| 3 | BD1008 | $-(CH_2)_4-$ | H | H | 0.34±0.07 |
| R-(+)-14 | LR174 | $-(CH_2)_4-$ | H | α-Me | 0.62±0.07 |
| S-(-)-14 | LR176 | $-(CH_2)_4-$ | H | β-Me | 0.83±0.17 |
| (±)-15 | BD1053 | Me, Me | Me | H | 1.46±0.17 |

Table 5: Effect of varying the distance between the 3,4-dichlorophenyl and N-methyl moieties as well as the pyrrolidinyl and N-methy groups on sigma receptor binding affinity

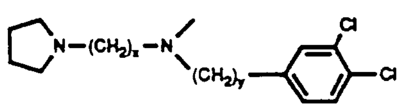

| Compound | Code | x | y | $K_i$ (nM) ([$^3$H]-(+)-3-PPP) |
|---|---|---|---|---|
| 3 | BD1008 | 2 | 2 | 0.34±0.07 |
| 16 | BD1078 | 3 | 2 | 1.76±0.02 |
| 17 | BD1186 | 2 | 1 | 0.38±0.03 |

**Table 6**: Effect of ring-size on the sigma receptor binding affinity of N-[2-(3,4-dichlorophenyl)ethyl]-N-methyl-2-(1-cycloalkyamine) ethylamines

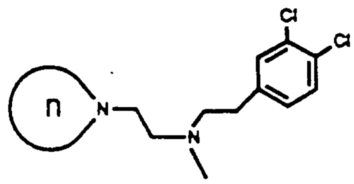

| Compound | Code | n | $K_i$ (nM) ([$^3$H]-(+)-3-PPP) |
|---|---|---|---|
| 3 | BD1008 | 5 | 0.34±0.07 |
| 18 | BD1072 | 6 | 0.26±0.07 |
| 19 | LR172 | 7 | 0.17±0.03 |

**Table 7**: Effect of aromatic substitution on the sigma receptor binding affinity of **3**

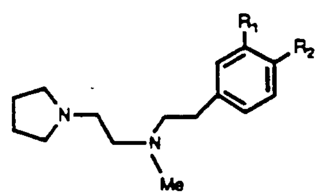

| Compound | Code | $R_1$ | $R_2$ | $K_i$ (nM) ([$^3$H]-(+)-3-PPP) |
|---|---|---|---|---|
| 20 | BD1192 | H | H | 7.40±0.14 |
| 21 | BD1190 | Cl | H | 0.84±0.03 |
| 22 | BD1188 | H | Cl | 1.47±0.08 |
| 3 | BD1008 | Cl | Cl | 0.34±0.07 |
| 23 | BD1196 | $R_1$,$R_2$=benzo | | 2.09±0.24 |

**Table 8**: Sigma ([$^3$H]-(+)-3-PPP), kappa ([$^3$H]bremazocine), PCP ([$^3$H]TCP) and $D_2$-dopamine ([$^3$H]sulpiride) receptor binding of selected compounds from Table **2-7**.

| Compound | Code | $K_i$ (nM) σ [$^3$H](+)PPP | κ [$^3$H]brem | PCP [$^3$H]TCP | $D_2$-Dopamine [$^3$H]Sulpiride |
|---|---|---|---|---|---|
| 3 | BD1008 | 0.34±0.07 | no inhibn[a] | no inhibn[a] | 1112±74 |
| 5 | BD1067 | 0.51±0.08 | no inhibn[a] | no inhibn[a] | 1665±69 |
| 11 | BD1120 | 0.73±0.11 | no inhibn[a] | no inhibn[a] | 3006±322 |
| R-(+)-14 | LR174 | 0.62±0.07 | no inhibn[a] | 3042±716 | 11235±161 |
| 17 | BD1186 | 0.38±0.03 | no inhibn[a] | no inhibn[a] | 1301±221 |
| 18 | BD1072 | 0.26±0.07 | no inhibn[a] | no inhibn[a] | 1209±15 |
| 19 | LR172 | 0.17±0.03 | no inhibn[a] | 6943±1046 | 6752±836 |
| 21 | BD1190 | 0.84±0.03 | no inhibn[a] | no inhibn[a] | 6497±322 |

## Biological Materials and Methods

### Membrane Preparation

Receptor binding assays were performed using the crude synaptosomal ($P_2$) membrane fraction of guinea pig brain (σ, κ, and PCP receptors) or rat brain (dopamine-$D_2$ receptors).

Crude $P_2$ membrane fractions were prepared from frozen (-80 °C) guinea pig brains (Pel-Freeze, Rogers, AK), minus cerebella. After removal of cerebella, brains were allowed to thaw slowly on ice and placed in ice-cold 10 mM Tris-HCl, pH 7.4 containing 320 mM sucrose (Tris-sucrose buffer). Brains were then homogenized in a Potter-Elvehjem homogenizer by 10 strokes of a motor driven Teflon pestle in a volume of 10 mL/gm tissue wet weight. The homogenate was centrifuged at 1,000 x g for 10 min at 4 °C and the supernatants saved. The pellets were resuspended by vortexing in 2 mL/g ice cold Tris-sucrose and centrifuged again at 1000 x g for 10 min. The combined 1000 x g supernatant was centrifuged at 31,000 x g for 15 min at 4 °C. The pellets were resuspended by vortexing in 3 mL/gm of 10 mM Tris-HCl, pH 7.4 and the suspension allowed to incubate at 25 °C for 15 min. Following centrifugation at 31,000 x g for 15 min, the pellets were resuspended by gentle Potter-Elvehjem homogenization to a final volume of 1.53 mL/gm in 10 mM Tris-HCl pH 7.4. Aliquots were stored at -80 °C until use. Protein concentration was determined by the method of Lowry et al.[26] using bovine serum albumin (BSA) as standard.

To prepare rat brain crude $P_2$ membranes, male Sprague-Dawley rats (150-200 g, Charles River, Boston, MA) were killed by decapitation. Brains (minus cerebella) were then treated as described above.

### Receptor Binding Assays

### Sigma Receptors

Sigma receptors were labeled with [$^3$H]-(+)-3-PPP (109 Ci/mmol). Incubations were carried out in 50 mM Tris-HCl, pH 8.0, for 120 min at 25 °C in a volume of 0.5 mL with 500 μg of membrane protein and 3 nM [$^3$H]-(+)-3-PPP. Nonspecific binding was determined in the presence of 1 μM haloperidol. Assays were terminated by the addition of 5 mL of ice-cold 10 mM Tris-HCl, pH 8.0, and filtration through glass fiber filters (Schleicher and Schuell). Filters were then washed twice with 5 mL of ice-cold Tris-HCl buffer. Filters were soaked in 0.5% polyethyleneimine for at least 30 min at 25 °C prior to use.

### Kappa Opiate Receptors

Kappa receptors were labelled with [3H]bremazocine (17.3 Ci/mmol) in the presence of [D-ala$^2$,-N-methyl-Phe$^4$,Gly-ol$^5$]-enkephalin (DAGO) and [D-Ser$^2$, Leu$^5$, Thr$^6$]-enkephalin (DSTLE) as $\mu$ and $\delta$ opiate receptor blockers, respectively. Incubations were carried out in 0.5 mL of 10 mM Tris-HCl, pH 7.4 for 90 min at 25 °C with 500 $\mu$g of membrane protein, 100 nM DAGO, 100 nM DSTLE, and 2 nM [$^3$H]-bremazocine. Assays were terminated by the addition of 5 mL of ice-cold buffer and filtration through glass fiber filters (Schleicher and Schuell) under reduced pressure. Filters were then washed twice with 5 mL ice-cold buffer. Nonspecific binding was determined in the presence of 10 $\mu$M levallorphan.

**Phencyclidine (PCP) Receptors.**

PCP receptors were labelled using [$^3$H]1-(1-(2-thienyl)cyclohexyl]piperidine ([$^3$H]TCP) (48.9 Ci/mmol). Incubations were carried out in 5 mM Tris-HCl, pH 7.4 for 60 min at 4 °C in a volume of 0.5 mL with 500 $\mu$g of membrane protein and 5 nM [$^3$H]TCP. Assays were terminated by addition of 5 mL of ice-cold buffer and filtration through glass fiber filters under reduced pressure. Filters were then washed twice with 5 mL ice-cold buffer. Filters were soaked in 0.3% polyethyleneimine for at least 30 min at 25 °C prior to use. Nonspecific binding was determined in the presence of 10 $\mu$M cyclazocine.

**Dopamine-$D_2$ Receptors**

Dopamine-$D_2$ receptors were labeled with 5 nM [$^3$H](-)-sulpiride (73.1 Ci/mmol) using rat brain membranes. Incubations were carried out for 60 min at 25 °C in 0.5 mL of 50 mM Tris-HCl, pH 7.7, containing 120 mM NaCl and 500 $\mu$g of membrane protein. Nonspecific binding was determined in the presence of 10 $\mu$M haloperidol. Assays were terminated by the addition of 5 mL of ice-cold incubation buffer and vacuum filtration through glass fiber filters (Schleicher and Schuell). Filters were then washed twice with ice-cold incubation buffer.

**Chemicals**

All scintillation counting was performed with a Packard model 4450 scintillation spectrometer using Ecoscint cocktail (National Diagnostics, Manville, NJ) after overnight extraction of the counts from the filters. Radioligands were purchased from DuPont/New England Nuclear (Boston, MA). Haloperidol, polyethyleneimine and Tris were purchased from Sigma Chemicals (St. Louis, MO). Cyclazocine and levallorphan were obtained from the National Institute on Drug Abuse (Rockville, MD).

**Legend to Table 1**

[a] all crystallizations were performed in an approx 1:10 weight/volume ratio of salt to solvent; [b] all yields are non-optimized; [c] concentrated aqueous HBr (47-48%) and concentrated aqueous HCl were utilized for formation of all the HBr and HCl salts in this Table; [d] $[\alpha]D = +10°$ (c 0.64, $H_2O$); [e] $[\alpha]D = -11.5°$ (c 0.77, $H_2O$); [f] compound reported in lit [23] but no physical data is given; [g] lit[23] mp for oxalate salt 152-155 °C; [h] base bp 49°/1.3mmHg; [i] base bp 56°/0.7mmHg; [j] reaction required greater than 24h at room temperature for completion; [k] base bp 73°/0.8mmHg; [l] base bp 88°/0.8mmHg; [m] $M^+$ (calc for $C_{12}H_{22}N_2O_3$): 242.1630, M+ (found): 242.1658; [n] $[\alpha]D = -27.6°$ (c 2.29, MeOH); [o] $M^+$ (calc for $C_{12}H_{22}N_2O_3$): 242.1630, M+ (found): 242.1618; [p] $[\alpha]D = +29.3°$ (c 1.34, MeOH); [q] $[\alpha]D = -12°$ (c 0.61, $H_2O$), compound is reported in lit [23] but no physical data is given; [r] $[\alpha]D = +15°$ (c 0.58, $H_2O$), compound is reported in lit [23] but no physical data is given; [s] $[\alpha]D = -33°$ (c 0.56, $H_2O$), lit[23] on HCl salt $[\alpha]D = -55.7°$ (c 1.0, $CHCl_3$); [t] lit [23] mp on HCl salt = 173-174 °C; [u] $[\alpha]D = +37°$ (c 0.59, $H_2O$), lit[23] on HCl salt $[\alpha]D = +56.5°$ (c 1.0, $CHCl_3$); [v] lit [23] mp on HCl salt 174-176 °C; [w] this corresponds to the product distribution of 8.2:1.8 of (±)-**57** to (±)-**58**; [x] $M^+$ (calc for $C_{13}H_{24}N_2O_3$): 256.1787 M+ (found): 256.1790; [y] base bp 64°/1.3mmHg; [z] $M^+$ (calc for $C_{17}H_{27}N_3O$): 289.2154, $M^+$ (found): 289.2145; as $M^+$ (calc for $C_{17}H_{27}N_3O$): 289.2154, $M^+$ (found): 289.2144.

**Legend to Tables 2-7**

Each compound was initially screened at concentrations of 10, 100, and 1000 nM in order to obtain an estimate of sigma binding affinity and to determine the appropriate concentration range to use in twelve-point concentration curves. For most compounds in the study, a concentration range of 0.0005-100 nM was appropriate. A range of 0.005-1000 nM or 0.05-10,000 nM was used for the less potent compounds. Twelve concentrations of unlabelled ligand were incubated with 3 nM [$^3$H](+)-3-PPP as described in Methods. The

CDATA iterative curve fitting program (EMF Software, Inc., Baltimore, MD) was used to determine $IC_{50}$ values. Values are the average of 2-4 experiments, ±SEM. Each experiment was carried out in duplicate. The Cheng-Prussoff equation[27] was then used to convert $IC_{50}$ values to apparent $K_i$ values. The $K_d$ for [$^3$H]-(+)-3-PPP (27.4 nM) was determined in independent experiments using guinea pig brain membranes.

**Legend to Table 8**

In order to obtain an initial estimate of binding affinity, three concentrations of each compound (100, 1000, and 10,000 nM) were incubated with the indicated radioligand for dopamine-$D_2$, kappa opiate or PCP receptors. Assay conditions were as described in Methods. Compounds eliciting > 30% inhibition were investigated further using twelve concentrations of unlabelled ligand ranging from 0.5-100,000 nM. Data were analyzed as described in the Legend of Tables 2-7. Values are the average of 2 experiments ± SEM., each carried out in duplicate. The following $K_d$ values (as determined in independent experiments) were employed to calculate $K_i$: [$^3$H](-)-Sulpiride (rat brain), $K_d$ = 10.3 nM; [$^3$H]Bremazocine (guinea pig brain), $K_d$ = 0.64 nM; [$^3$H]TCP (guinea pig brain), $K_d$ = 25 nM. [a] No $IC_{50}$ or $K_i$ value was determined since the compound produced less than 30% inhibition of control binding at a concentration of 10,000 nM. Sigma receptor binding data are taken from Tables 2-7.

**Table of elemental analyses**

| Compound | Mol. | | % Calcd | | | % Found | |
|---|---|---|---|---|---|---|---|
| | Formula | C | H | N | C | H | N |
| **3**.HBr | $C_{15}H_{24}Br_2Cl_2N_2$ | 38.91 | 5.22 | 6.05 | 38.99 | 5.24 | 5.97 |
| **4**.HBr | $C_{14}H_{22}Br_2Cl_2N_2$ | 37.45 | 4.94 | 6.24 | 37.56 | 4.93 | 6.16 |
| **5**.HBr | $C_{16}H_{26}Br_2Cl_2N_2$ | 40.28 | 5.49 | 5.87 | 40.43 | 5.55 | 5.95 |
| **6**.HBr | $C_{17}H_{28}Br_2Cl_2N_2$ | 41.57 | 5.75 | 5.70 | 41.53 | 5.78 | 5.70 |
| **7**.HBr | $C_{17}H_{26}Br_2Cl_2N_2$ | 41.75 | 5.36 | 5.73 | 41.81 | 5.36 | 5.71 |
| **8**.oxalate | $C_{22}H_{32}Cl_2N_2O_8$ | 50.48 | 6.16 | 5.35 | 50.73 | 6.41 | 5.52 |
| **9**.HBr | $C_{13}H_{22}Br_2Cl_2N_2$ | 35.73 | 5.07 | 6.41 | 35.81 | 5.06 | 6.43 |
| **10**.HBr | $C_{12}H_{20}Br_2Cl_2N_2 \cdot H_2O$ | 32.68 | 5.03 | 6.35 | 32.85 | 5.00 | 6.38 |
| **11**.HBr | $C_{15}H_{26}Br_2Cl_2N_2$ | 38.74 | 5.63 | 6.02 | 38.83 | 5.63 | 6.05 |
| **12**.HBr | $C_{12}H_{20}Br_2Cl_2N_2$ | 34.07 | 4.77 | 6.62 | 33.95 | 4.76 | 6.63 |
| **13**.HBr | $C_{10}H_{16}Br_2Cl_2N_2$ | 30.41 | 4.08 | 7.09 | 30.51 | 4.06 | 7.10 |
| **(R)-14**.oxalate | $C_{20}H_{28}Cl_2N_2O_8$ | 48.49 | 5.70 | 5.66 | 48.27 | 5.69 | 5.60 |
| **(S)-14**.oxalate | $C_{20}H_{28}Cl_2N_2O_8$ | 48.49 | 5.70 | 5.66 | 48.57 | 5.75 | 5.66 |
| **(±)-15**.oxalate | $C_{18}H_{26}Cl_2N_2O_8$ | 46.07 | 5.58 | 5.97 | 45.94 | 5.62 | 6.02 |
| **16**.HBr | $C_{16}H_{26}Br_2Cl_2N_2$ | 40.28 | 5.49 | 5.87 | 40.41 | 5.52 | 5.93 |
| **17**.HCl | $C_{14}H_{22}Cl_4N_2$ | 46.69 | 6.16 | 7.78 | 46.60 | 6.17 | 7.77 |
| **18**.HBr | $C_{16}H_{26}Br_2Cl_2N_2$ | 40.28 | 5.49 | 5.87 | 40.37 | 5.54 | 5.83 |
| **19**.HCl | $C_{17}H_{28}Cl_4N_2$ | 50.76 | 7.02 | 6.96 | 50.81 | 7.03 | 6.95 |
| **20**.HCl | $C_{15}H_{26}Cl_2N_2$ | 59.01 | 8.58 | 9.18 | 59.07 | 8.59 | 9.14 |
| **21**.HCl | $C_{15}H_{25}Cl_3N_2$ | 53.03 | 7.42 | 8.25 | 53.04 | 7.46 | 8.21 |
| **22**.HCl | $C_{15}H_{25}Cl_3N_2$ | 53.03 | 7.42 | 8.25 | 53.10 | 7.44 | 8.21 |
| **23**.HBr | $C_{19}H_{28}Br_2N_2$ | 51.37 | 6.35 | 6.31 | 51.40 | 6.36 | 6.30 |
| **25**.oxalate | $C_{11}H_{20}N_2O_8$ | 42.86 | 6.54 | 9.09 | 42.99 | 6.51 | 9.17 |
| **27**.HCl | $C_{15}H_{21}Cl_3N_2O$ | 51.23 | 6.02 | 7.96 | 51.29 | 6.05 | 7.94 |
| **28**.fumarate | $C_{14}H_{22}N_2O_8$ | 48.55 | 6.40 | 8.09 | 48.64 | 6.42 | 8.03 |
| **29**.oxalate | $C_{12}H_{22}N_2O_8$ | 44.72 | 6.88 | 8.69 | 44.80 | 6.87 | 8.68 |

**Table of elemental analyses continued**

| Compound | Mol. Formula | % Calcd C | H | N | % Found C | H | N |
|---|---|---|---|---|---|---|---|
| **30**.HCl | $C_9H_{22}Cl_2N_2$ | 47.17 | 9.68 | 12.22 | 47.08 | 9.65 | 12.18 |
| **31**.fumarate | $C_{17}H_{26}N_2O_8$ | 52.84 | 6.78 | 7.25 | 52.74 | 6.78 | 7.24 |
| **32**.fumarate | $C_{18}H_{30}N_2O_8$ | 53.72 | 7.51 | 6.96 | 53.66 | 7.51 | 6.94 |
| **33** (base) | $C_{14}H_{18}Cl_2N_2O$ | 55.83 | 6.02 | 9.30 | 55.79 | 6.02 | 9.27 |
| **34**.oxalate | $C_{18}H_{24}Cl_2N_2O_5$ | 51.56 | 5.77 | 6.68 | 51.56 | 5.81 | 6.62 |
| **35**.oxalate | $C_{19}H_{26}Cl_2N_2O_5$ | 52.66 | 6.05 | 6.46 | 52.74 | 6.06 | 6.45 |
| **36**.fumarate | $C_{21}H_{26}Cl_2N_2O_5$ | 55.15 | 5.73 | 6.13 | 55.10 | 5.75 | 6.09 |
| **37**.oxalate | $C_{20}H_{28}Cl_2N_2O_5$ | 53.70 | 6.31 | 6.26 | 53.62 | 6.34 | 6.24 |
| **38**.oxalate | $C_{17}H_{24}N_2O_5$ | 60.70 | 7.19 | 8.33 | 60.63 | 7.22 | 8.29 |
| **39**.oxalate | $C_{17}H_{23}ClN_2O_5$ | 55.06 | 6.25 | 7.55 | 55.06 | 6.30 | 7.50 |
| **40**.oxalate | $C_{17}H_{23}ClN_2O_5$ | 55.06 | 6.25 | 7.55 | 55.16 | 6.26 | 7.57 |
| **41**.fumarate | $C_{23}H_{28}N_2O_5$ | 66.97 | 6.84 | 6.79 | 67.00 | 6.86 | 6.77 |
| **41a** (base) | $C_{12}H_{16}Cl_2N_2O$ | 52.38 | 5.86 | 10.18 | 52.31 | 5.86 | 10.13 |
| **42a**.HCl | $C_{13}H_{19}Cl_3N_2O \cdot 0.5H_2O$ | 46.66 | 6.02 | 8.37 | 46.77 | 5.86 | 8.49 |
| **44** fumarate | $C_{13}H_{22}N_2O_8$ | ***46.70*** | ***6.63*** | ***8.38*** | ***45.91*** | ***6.22*** | ***7.23*** |
| **46**.fumarate | $C_{15}H_{26}N_2O_8$ | 49.72 | 7.23 | 7.73 | 49.83 | 7.28 | 7.72 |
| **47**.oxalate | $C_{17}H_{24}Cl_2N_2O_5$ | 50.13 | 5.94 | 6.88 | 50.17 | 5.96 | 6.87 |
| **50**.oxalate | $C_{14}H_{18}Cl_2N_2O_5$ | 46.04 | 4.97 | 7.67 | 45.86 | 5.04 | 7.61 |
| **51**.fumarate | $C_{14}H_{16}Cl_2N_2O_5$ | 46.30 | 4.44 | 7.71 | 46.44 | 4.49 | 7.75 |
| **(S)-53** | $C_{12}H_{22}N_2O_3$ | 59.48 | 9.15 | 11.56 | 59.30 | 9.18 | 11.62 |
| **(R)-53** | $C_{12}H_{22}N_2O_3$ | 59.48 | 9.15 | 11.56 | 59.27 | 9.23 | 11.50 |
| **(S)-54**.oxalate | $C_{12}H_{22}N_2O_8$ | 44.72 | 6.88 | 8.69 | 44.57 | 6.86 | 8.67 |
| **(R)-54**.oxalate | $C_{12}H_{22}N_2O_8$ | 44.72 | 6.88 | 8.69 | 44.73 | 6.46 | 8.42 |
| **(S)-55**.oxalate | $C_{18}H_{24}Cl_2N_2O_5.H_2O$ | 49.44 | 5.99 | 6.41 | 44.37 | 5.07 | 5.55 |
| **(R)-55**.oxalate | $C_{18}H_{24}Cl_2N_2O_5.H_2O$ | 49.44 | 5.99 | 6.41 | 49.59 | 5.65 | 6.40 |
| **(±)-57**.oxalate | $C_{10}H_{20}N_2O_8$ | 40.54 | 6.80 | 9.46 | 40.46 | 6.81 | 9.50 |
| **(±)-60**.oxalate | $C_{16}H_{22}Cl_2N_2O_5$ | 48.87 | 5.64 | 7.12 | 48.91 | 5.66 | 7.09 |

**Table of elemental analyses continued**

| Compound | Mol. Formula | % Calcd C | H | N | % Found C | H | N |
|---|---|---|---|---|---|---|---|
| 62.fumarate | $C_{16}H_{26}N_2O_8$ | *51.33* | *7.00* | *7.48* | *50.15* | *7.08* | *7.29* |
| 63.oxalate | $C_{18}H_{24}Cl_2N_2O_5$ | 51.56 | 5.77 | 6.68 | 51.59 | 6.16 | 6.26 |
| 66.oxalate | $C_{13}H_{24}N_2O_8$ | 46.42 | 7.19 | 8.33 | 45.70 | 6.92 | 8.12 |
| 67.oxalate | $C_{19}H_{26}Cl_2N_2O_5.0.5H_2O$ | 51.88 | 6.11 | 6.33 | 51.29 | 6.10 | 5.89 |
| 69.oxalate | $C_{10}H_{18}N_2O_5$ | 48.77 | 7.37 | 11.38 | 48.80 | 7.45 | 11.44 |
| 70.fumarate | $C_{16}H_{26}N_2O_8$ | 51.33 | 7.00 | 7.48 | 51.28 | 7.05 | 7.44 |
| 71.fumarate | $C_{20}H_{26}Cl_2N_2O_5$ | *53.94* | *5.88* | *6.29* | *51.43* | *5.77* | *6.65* |
| 72.fumarate | $C_{18}H_{22}Cl_2N_2O_5$ | 51.81 | 5.31 | 6.71 | 51.90 | 5.34 | 6.76 |

The following Examples are detailed descriptions of the methods of preparation of compounds of Formula I. These detailed preparations fall within the scope of, and serve to exemplify, the above described Generic Procedures which form part of the invention. These Examples are presented for illustrative purposes only and are not intended as a restriction on the scope of the invention. All parts are by weight unless otherwise indicated. Most of the commercially available starting materials were obtained from Aldrich Chemical Company, Milwaukee, Wisconsin. Abbreviated terms used in Examples are explained below:

| | |
|---|---|
| MeOH = | methanol |
| EtOH = | ethanol |
| DMSO = | dimethylsulfoxide |
| EtOAc = | ethyl acetate |
| TLC = | thin layer chromatography |
| $Et_2O$ = | ethyl ether |
| $Ac_2O$ = | acetic anhydride |
| $Et_3N$ = | triethylamine |
| THF = | tetrahydrofuran |

## Table 1: PHYSICAL AND σ BINDING DATA

| Compound Number | Struct. | Ki ([$^3$H](+)-3-PPP)[a] | mp (°C) | Anal (C, H, N)% m.f. calc fnd |
|---|---|---|---|---|
| BD-1060 | 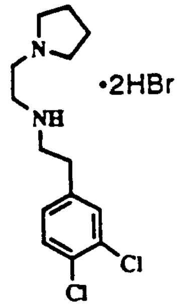 •2HBr | 1.11±0.11 | 244-245 (dec) | $C_{14}H_{22}Br_2Cl_2N_2$<br>37.45 4.94 6.24<br>37.56 4.93 6.16 |
| BD-1008 | 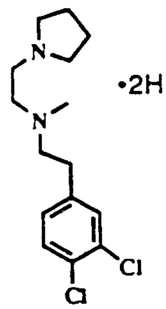 •2HBr | 0.34±0.07 | 264-265 (dec) | $C_{15}H_{24}Br_2Cl_2N_2$<br>38.91 5.22 6.05<br>38.99 5.24 5.97 |
| BD-1067 | 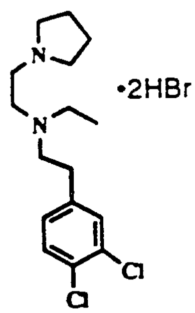 •2HBr | 0.51±0.08 | 188-189 | $C_{16}H_{26}Br_2Cl_2N_2$<br>40.28 5.49 5.87<br>40.43 5.55 5.95 |

| | | | |
|---|---|---|---|
| BD-1121 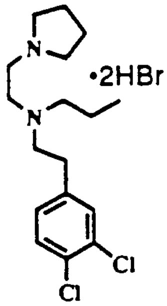 •2HBr | 1.35±0.15 | 190-191 | $C_{17}H_{28}Br_2Cl_2N_2$<br>41.57 5.75 5.70<br>41.53 5.78 5.70 |
| BD-1052 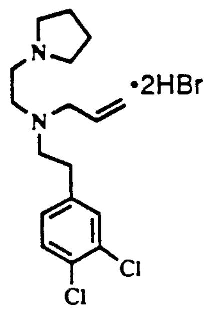 •2HBr | 1.39±0.03 | 178-179 | $C_{17}H_{26}Br_2Cl_2N_2$<br>41.75 5.36 5.73<br>41.81 5.36 5.71 |
| BD-1069 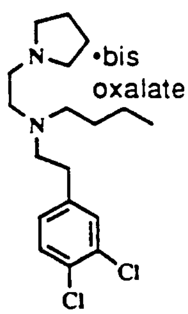 •bis oxalate | 11.7±2.5 | 203-204 (dec) | $C_{22}H_{32}Cl_2N_2O_8$<br>50.48 6.16 5.35<br>50.73 6.41 5.52 |

| | | | | |
|---|---|---|---|---|
| LR-174[b] | 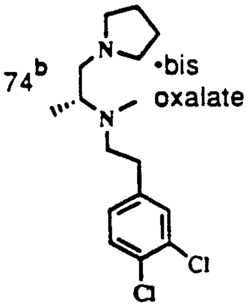 •bis oxalate | 0.62±0.07 | 198-200 | $C_{20}H_{28}Cl_2N_2O_8$<br>48.49 5.70 5.66<br>48.27 5.69 5.60 |
| LR-176[c] | 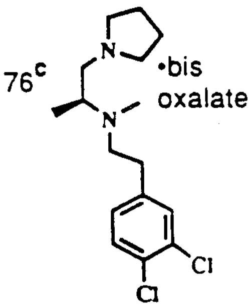 •bis oxalate | 0.83±0.17 | 198-200 | $C_{20}H_{28}Cl_2N_2O_8$<br>48.49 5.70 5.66<br>48.57 5.75 5.66 |

| | | | | |
|---|---|---|---|---|
| BD-1078 | 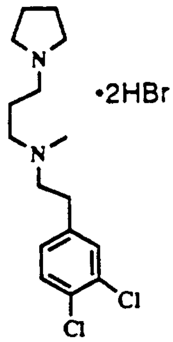 •2HBr | 1.76±0.02 | 269-270 (dec) | $C_{16}H_{26}Br_2Cl_2N_2$<br>40.28 5.49 5.87<br>40.41 5.52 5.93 |
| BD-1186 | 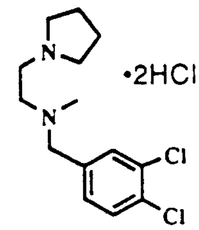 •2HCl | 0.38±0.03 | 258-259 (dec) | $C_{14}H_{22}Cl_4N_2$<br>46.69 6.16 7.78<br>46.60 6.17 7.77 |
| BD-1072 | 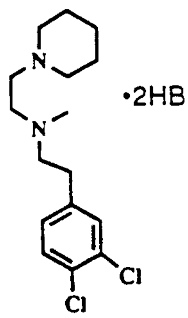 •2HBr | 0.26±0.07 | 264-265 (dec) | $C_{16}H_{26}Br_2Cl_2N_2$<br>40.28 5.49 5.87<br>40.37 5.54 5.83 |
| LR-172 | 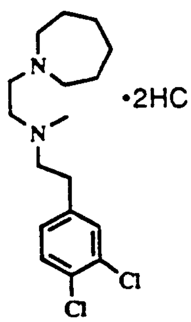 •2HCl | 0.17±0.03 | 260.5-261.5 (dec) | $C_{17}H_{28}Cl_4N_2$<br>50.76 7.02 6.96<br>50.81 7.03 6.95 |

| | | | | | |
|---|---|---|---|---|---|
| BD-1192 | 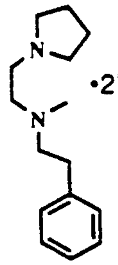 | •2HCl | 7.40±0.14 | 266-267 (dec) | $C_{15}H_{26}Cl_2N_2$<br>59.01 8.58 9.18<br>59.07 8.59 9.14 |
| BD-1190 | 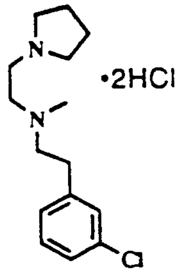 | •2HCl | 0.84±0.03 | 258-259 (dec) | $C_{15}H_{25}Cl_3N_2$<br>53.03 7.42 8.25<br>53.04 7.46 8.21 |
| BD-1188 | 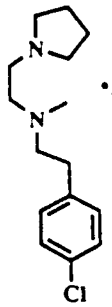 | •2HCl | 1.47±0.08 | 257-258 (dec) | $C_{15}H_{25}Cl_3N_2$<br>53.03 7.42 8.25<br>53.10 7.44 8.21 |
| BD-1196 | 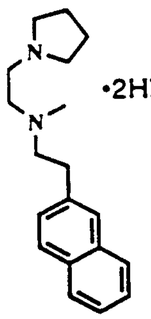 | •2HBr | 2.09±0.24 | 259-260 (dec) | $C_{19}H_{28}Br_2N_2$<br>51.37 6.35 6.31<br>51.40 6.36 6.30 |

Biological Evaluation

Compounds of the invention were evaluated by one or more of the following procedures:

[a] The Ki binding data for Table 1 was generated as described in the Methods section (see below). In order to obtain an initial estimate of the binding affinity of the compounds, each compound was initially tested at 3 concentrations in the $\sigma$ assay (100, 1000, 10000 nM). If a compound elicited > 30% inhibition of binding of the test ligand ([3H]-(+)-3-PPP) at 10000 nM, a twelve point curve using unlabeled test ligand concentrations ranging from 0.05-10000 nM (0.005-1000 nM for the most potent compounds) was conducted. The CDATA (EMF Software, Inc., Baltimore, MD) iterative curve fitting program was used to analyze the results. The above results are the results of two, or in some cases three experiments (± SEM), each carried out in duplicate.

[b] $[\alpha]D = +10^o$ (c 0.64, $H_2O$)
[c] $[\alpha]D = -11.5^o$ (c 0.77, $H_2O$)

Receptor binding assays were performed using the crude synaptosomal ($P_2$) membrane fraction of guinea pig brain for sigma receptors.

Crude $P_2$ membrane fractions were prepared from frozen (-80 °C) guinea pig brains (Pel-Freeze, Rogers, AK), minus cerebellum. After removal of cerebella, brains were allowed to thaw slowly on ice and placed in ice-cold 10 mM Tris-HCl, pH 7.4 containing 320 mM sucrose (Tris-sucrose buffer). Brains were then homogenized in a Potter-Elvehjem homogenizer by 10 strokes of a motor driven Teflon pestle in a volume of 10 mL/gm tissue wet weight. The homogenate was centrifuged at 1,000 x g for 10 min at 4 °C and the supernatants saved. The pellets were resuspended by vortexing in 2 mL/g ice cold Tris-sucrose and centrifuged again at 1000 x g for 10 min. The combined 1000 x g supernatant was centrifuged at 31,000 x g for 15 min at 4 °C. The pellets were resuspended by vortexing in 3 mL/gm of 10 mM Tris-HCl, pH 7.4 and the suspension allowed to incubate at 25 °C for 15 min. Following centrifugation at 31,000 x g for 15 min, the pellets were resuspended by gentle Potter-Elvehjem homogenization to a final volume of 1.53 mL/gm in 10 mM Tris-HCl pH 7.4. Aliquots were stored at -80 °C until use. Protein concentration was determined by the method of Lowry et al using bovine serum albumen as standard.

$\sigma$ receptors were labelled with $[^3H]$-(+)-3-PPP (98.9 Ci/mmol). Incubations were carried out in 50 mM Tris-HCl, pH 8.0, for 120 min at 25 °C in a volume of 0.5 mL with 500 $\mu$g of membrane protein and 3 nM $[^3H]$-(+)-3-PPP. Nonspecific binding was determined in the presence of 1 $\mu$M haloperidol. Assays were terminated by the addition of 5 mL of ice-cold 10 mM Tris-HCl, pH 8.0, and filtration through glass fiber filters (Schleicher and Schuell), which were soaked in 0.5% poly(ethylenimine) for at least 30 min at 25 °C prior to use. Filters were then washed twice with 5 mL of ice-cold Tris-HCl buffer.

## Blockage of Apomorphine-induced Climbing

Compounds of the invention can be evaluated for their ability to block apomorphine-induced climbing. The evaluation of the compounds follows the method outlined by Protais [Psychopharmacol., 50, 1-6, 1976]. Swiss-Webster mice, weighing 20-25 g, are pretreated with the compounds of the invention by i.p. or s.c. administration at various times before 2 mg/kg apomorphine is administered s.c. in a volume of 1 ml/kg. All test compounds are administered in a volume of 10 ml/kg. Mice are rated at 10 and 20 minutes after apomorphine administration using the following rating scale: (0) forepaws on the floor, (1) forefeet holding the bars, and (2) four paws holding bars. Dose-response curves are analyzed by a computerized Finney assay [Statistical Methods in Biological Assays, 2nd Edn., Hatner Pub. Co., New York (1964)]. Compounds of the invention would be expected to block apomorphine-induced climbing at a dose of 10 mg/kg.

## Forebrain Ischemia Assay

Male Mongolian gerbils, 50-70 gm, may be used as subjects. Compound of the invention (50 mg/kg) is injected i.p. 30 minutes prior to carotid occlusion into 6 gerbils. In preparation for surgical procedures, the animals are lightly anesthetized with halothane and placed upside down on a heated pad with their snout within a nosecone. Nitrous oxide (70%): oxygen (30%) plus 0.5% halothane is circulated through the nosecone to provide continuous anesthesia throughout the surgical procedure. A midline incision is made in the neck and the carotid arteries are exposed. A length of suture thread is placed under each carotid. The thread is then tightened around each carotid and pressure applied to the thread to insure flow is occluded. Flow is occluded for 15 minutes and then the thread is removed. The carotids are visually inspected to confirm that reflow occurs. The wound is then closed with autoclips and the gerbils are allowed to recover. Following surgery, the gerbils are kept alive for 7 days. They are anesthetized with 100 mg/kg sodium pentobarbital and perfused transcardially with saline (with heparin) followed by buffered formalin. The brain is removed, trimmed and prepared for histological processing. Sections (10 microns) are stained with thionin. At 7 days following the ischemic insult, damaged neurons are cleared away by glia and the extent of damage can be ascertained within the vulnerable CA1 region of the hippocampus. The degree of lesion in the CA1 region of the hippocampus is quantified by counting the pyramidal cell bodies in a 0.5 mm length of CA1 on the section corresponding to P 1.7 mm in the atlas of Loskota, Lomax and Verity [W. J. Loskota et al, A Stereotaxic Atlas of the Mongolian Gerbil Brain, Ann Arbor Science Publishers, Ann Arber, p. 77 (1974)]. The cell loss would be significantly reduced in the gerbils given a compound of Formula I.

## Circling Behavior Assay

In order to assess the utility of compounds of the invention in treatment of Parkinsonism, the ability of the compound to stimulate movement following microinjections in the substnatia nigra is assessed. Parkinson's disease results from the degeneration of dopamine-secreting neurons in the substantia nigra resulting in a reduced ability to generate movement. Compound of the invention binds to sigma receptors, which are highly concentrated in the substantia nigra. The demonstration of increased motor activity, following compound administration in this part of the brain, would therefore suggest a stimulatory action on the substantia nigra. Such a stimulatory action would predict increased ability to generate voluntary movement in Parkinson's patients showing decreased functionality of this brain area. Current treatments of Parkinson's disease also stimulate this brain area, but show significant side effects that may n ot be present in compounds of the invention. The following experiments demonstrate the ability of compounds of the invention to stimulate motor activity following microinjections in the substantia nigra. It is expected, therefore, that compounds of the invention would have clinical utility in the treatment of Parkinson's disease and other disorders in which there is a paucity of movement.

Three to seven days before behavioral testing, each animal (245-350 g, N = 57) is anesthetized with 50 mg/kg sodium pentobarbital and mounted in a Narishige stereotaxic apparatus. A guide cannula, constructed from 24 guage thinwall stainless steel tubing, is implanted with its tip 4.0 mm above the left substantia nigra pars reticulata (coordinates: 3.2 mm anterior; 2.2 mm lateral; 4.7 mm ventral), from lambda and the skull surface. Cannulae are secured with stainless steel screws and dental acrylic. Stainless steel stylets keep the cannulae sealed except during drug infusion. All drugs are prepared on the day of testing. Animals are gently restrained and the drugs are administered in a volume of 0.5 ul over 72 seconds through a 31 guage microneedle that is constructed to extend 4.0 mm beyond the tips of the guide cannulae. Animals receive a single injection of saline (n = 6), 1.5 nmol (n = 5), 3.7 nmol (n = 6), 9.3 nmol (n = 7 or 14.9 nmol (n = 5) in 0.5 ul 0.9% NaCl. Following injection, each animal is fitted with a harness which is connected to an optical position transducer interfaced to a computer that derives the number and direction of half turns for 30 min. Each rat receives a single injection of the test drug to minimize tissue damage. Rats are sacrificed by an overdose of sodium pentobarbital and perfused intracardially with 10% formalin. Brains are fixed in a 30% sucrose-formalin solution and coronal sections (40 um) are taken throughout the extent of the injection site. The sections are stained with cresyl violet and examined under a microscope to localize injection sites. Only subjects with histologically confirmed injection sites are used in the data analyses. For the studies of circling behavior, the number of net (contalateral-isolateral) rotations are computed for each rat and subjected to analysis of variance. Repeated measures analysis of variance would reveal that compound of the invention produce significant dose-related increases in circling behavior compared to the saline control following microinjectons in the substantia nigra ($F_{4,27} = 3.09$; $p = 0.032$). Consistent with the increased affinity of compound of the invention for sigma receptors, this effect occurrs at doses lower than those necessary to produce the effect with (+)-pentazocine published previously. Compounds of the invention would be expected to show contralateral circling behavior produced by intranigral microinjections of various doses of compound of the invention herein.

Neuroprotective properties of BD-1008 and selected compounds of this invention have been evaluated in the dynorphin A induced spinal cord injury model. Along with several other neuropeptides, dynorphin A has potent vasoconstrictory effects on the vasculature of the rat brain and spinal cord; thus it provides a convenient, reproducible pharmacological means to induce and study ischemic central nervous system injury. Furthermore, localized effects of this peptide on the lumbosacral spinal cord following L4-L5 intervertebral injection allows for simple and straightforward neurological appraisals through assessment of hindlimb motor function. Using this approach, we have determined that the potent sigma ligand BD-1008 significantly improves neurological recovery from the flaccid hindlimb paralysis resulting from exposure to a 20 nanomole dose of dynorphin A. The protective effects of this compound occurred without any measurable impact on the primary vascular responses to dynorphin A, indicating that the protection resulted from the interuption of secondary deleterious processes in the pathophysiological cascade set into motion by the dynorphin A-induced reduction in lumbosacral blood flow.

## Figure 1

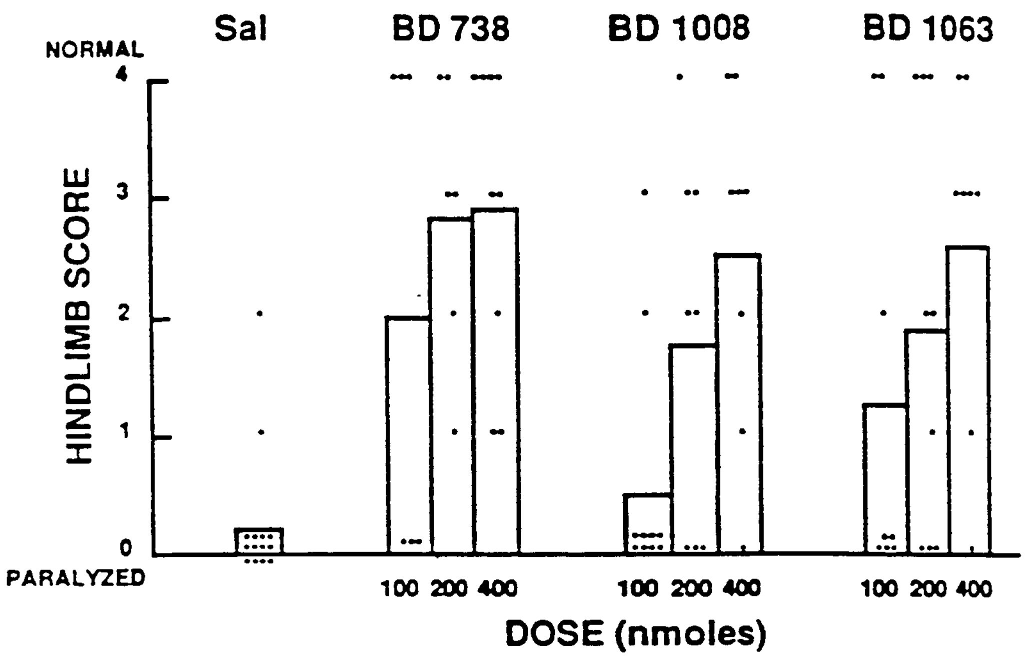


Legend for Figure 1: Dose-dependent protective effects of BD-738, BD-1008 and BD-1063 24 hours following spinal subarachnoid coinjection with a paralytic 20 nanomole dose of dynorphin A. These compounds did not prevent the immediate loss of hindlimb motor function, but did significantly improve its subsequent recovery relative to dynorphin A-treated rats coinjected with saline vehicle. Neurological scores assigned to individual rats are depicted as points, and mean scores for treatment groups are represented by bar heights. Neurological; scores are assigned as follows:

4 = normal motor function.
3 = paraparesis, with the ability to support weight and walk with impairment.
2 = paraparesis, with the ability to make walking movements without fully supporting weight.
1 = severe paraparesis, in which animals could make hindlimb movements but not walking movements.
0 = flaccid paralysis, with complete absence of any hindlimb movement.

Figure 2A

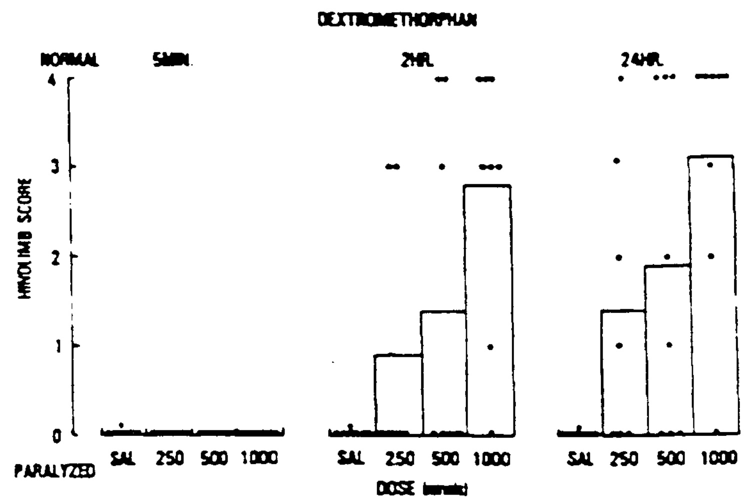


Figure 2B

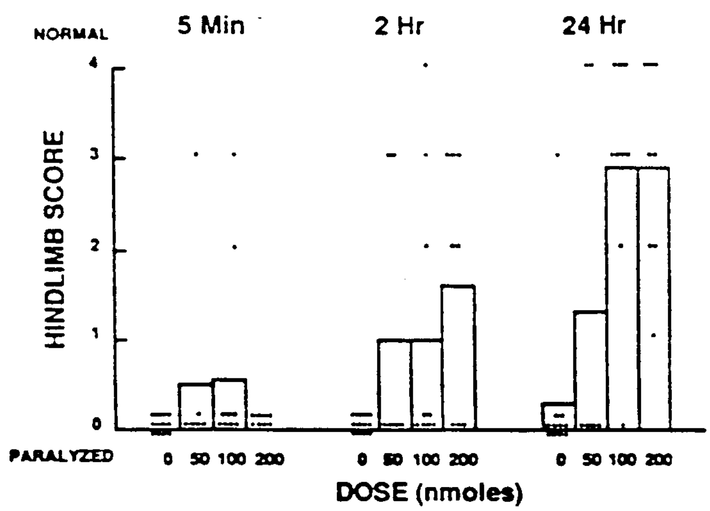


Legend for Figure 2A and 2B: Dose and time-dependent protective effects of the PCP-sigma receptor ligand dextromethorphan (A) and the specific sigma receptor ligand BD-737 (B). These compounds did not present the immediate loss of hindlimb motor function, but did improve its subsequent recovery. Neurological scores were assigned using the 5 point scale described in the preceeding figure legend.

Figure 2A

CHANGE IN LUMBOSACRAL BLOOD FLOW

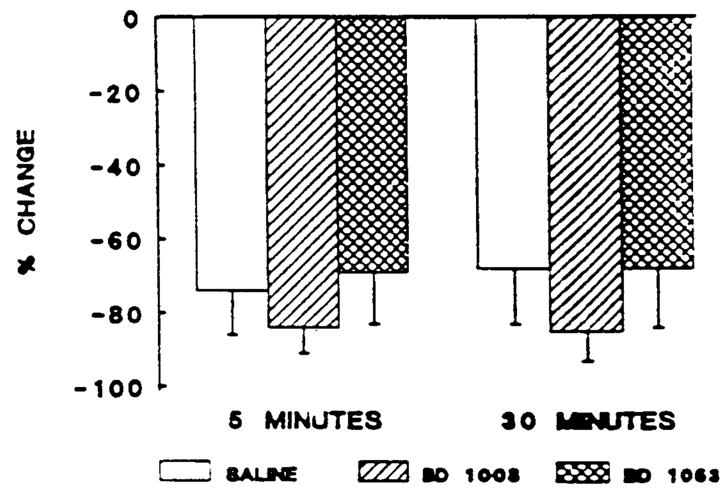


Figure 2B

CHANGE IN LUMBOSACRAL BLOOD FLOW

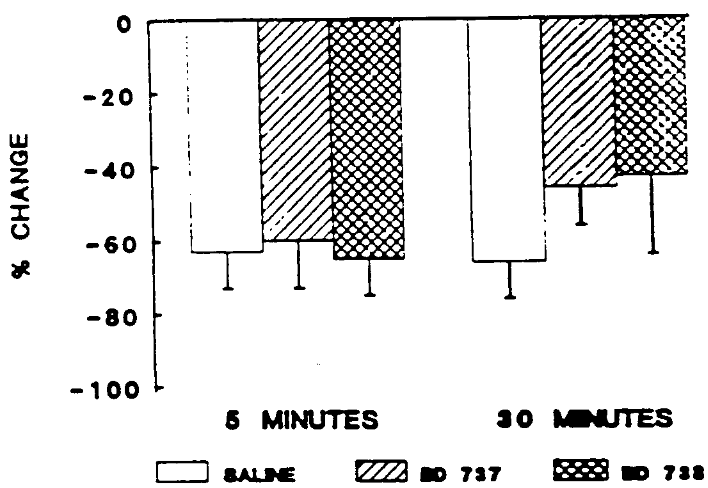


Legend for Figure 3A and 3B: Changes in lumbosacral blood flow 5 and 30 minutes following spinal subarachnoid injection of 40 nanomoles of dynorphin A in combination with: (A) saline vehicle, BD-1008 and BD1063 (400 nanomoles each) or (B) 50% DMSO vehicle, BD-737 and BD738 (400 nanomoles each). Data am presented as mean percentage changes (±S.E.M.) from pretreatment measurements made 10 minutes prior to subarachnoid injections. Baseline lumbosacral blood flows were 68 ± 5.2 ml/min/100 g and did not differ among treatment groups.

| Effects of Dynorphin A on CSF Lactic Acid Concentrations Following Drug Cotreatments | | |
|---|---|---|
| TREATMENT | LACTIC ACID[1] | N |
| Saline vehicle | 21.6±2.1 | 13 |
| Saline & Dynorphin A | 56.7±2.0* | 11 |
| BD 737 & Dynorphin A | 59.1±1.9* | 11 |
| BD 738 & Dynorphin A | 56.4± 3.3* | 9 |
| BD 1008 & Dynorphin A | 54.0±2.8* | 6 |
| BD 1063 & Dynorphin A | 64.2±1.8* | 6 |

[1]mg/dl
*$p<0.05$ when compared to saline-injected rats

Also embraced within this invention is a class of pharmaceutical compositions comprising one or more compounds of Formula I in association with one or more non-toxic, pharmaceutically-acceptable carriers and/or diluents and/or adjuvants (collectively referred to herein as "carrier" materials) and, if desired, other active ingredients. The compounds of the present invention may be administered by any suitable route, preferably in the form a pharmaceutical composition adapted to such a route, and in a dose effective for the treatment intended. Therapeutically effective doses of the compounds of the present invention required to prevent or arrest the progress of the medical condition are readily ascertained by one of ordinary skill in the art. The compounds and composition may, for example, be administered intravascularly, intraperitoneally, subcutaneously, intramuscularly or topically.

For oral administration, the pharmaceutical compositions may be in the form of, for example, a tablet, capsule, suspension or liquid. The pharmaceutical composition is preferably made in the form of a dosage unit containing a particular amount of the active ingredient. Examples of such dosage units are tablets or capsules. These may with advantage contain an amount of active ingredient from about 1 to 250 mg, preferably from about 25 to 150 mg. A suitable daily dose for a mammal may very widely depending on the condition of the patient and other factors. However, a dose of from about 0.1 to 3000 mg/kg body weight, particularly from about 1 to 100 mg/kg body weight, may be appropriate.

The active ingredient may also be administered by injection as a composition wherein, for example, saline, dextrose or water may be used as a suitable carrier. A suitable daily dose is from about 0.1 to 100 mg/kg body weight injected per day in multiple doses depending on the disease being treated. A preferred daily dose would be from about 1 to 30 mg/kg body weight. Compounds indicated for prophylactic therapy will preferably be administered in a daily dose generally in a range from about 0.1 mg to about 100 mg per kilogram of body weight per day. A more preferred dosage will be a range from about 1 mg to about 100 mg per kilogram of body weight. Most preferred is a dosage in a range from about 1 to about 50 mg per kilogram of body weight per day. A suitable dose can be administered, in multiple sub-doses per day. These sub-doses may be administered in unit dosage forms. Typically, a dose or sub-dose may contain from about 1 mg to about 100 mg of active compound per unit dosage form. A more preferred dosage will contain from about 2 mg to about 50 mg of active compound per unit dosage form. Most preferred is a dosage form containing from about 3 mg to about 25 mg of active compound per unit dose.

The dosage regimen for treating a disease condition with the compounds and/or compositions of this invention is selected in accordance with a variety of factors, including the type, age, weight, sex and medical condition of the patient, the severity of the disease, the route of administration, and the particular compound employed, and thus may vary widely.

For therapeutic purposes, the compounds of this invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

Although this invention has been described with respect to specific embodiments, the details of these embodiments are not to be construed as limitations. Various equivalents, changes and modifications may be made without departing from the spirit and scope of this invention, and it is understood that such equivalent embodiments are part of this invention.

## Claims

1. A compound of Formula I:

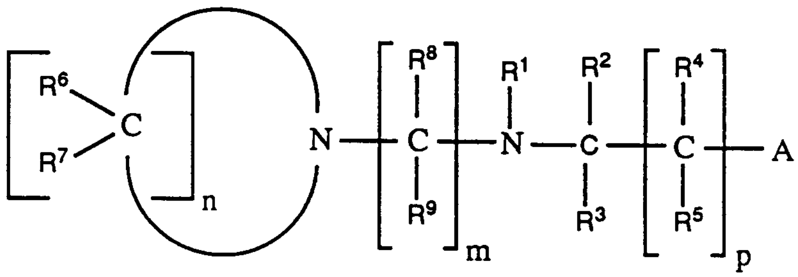

(I)

wherein $R^1$ is selected from hydrido, alkyl, cycloalkyl, hydroxyalkyl, haloalkyl, cycloalkylalkyl, alkoxyalkyl, aralkyl, aryl, alkenylalkyl, alkynylalkyl, carboxyalkyl, alkanoyl, alkylsulfinyl, alkylsulfonyl, arylsulfinyl and arylsulfonyl; wherein each of $R^2$ and $R^3$ is independently selected from hydrido, alkyl, cycloalkyl, hydroxyalkyl, haloalkyl, cycloalkylalkyl, alkoxyalkyl, aralkyl, aryl, alkenyl, alkynyl, alkenylalkyl, alkynylalkyl, carboxyalkyl, alkanoyl, alkoxycarbonyl, carboxy, cyanoalkyl, alkylsulfinyl, alkylsulfonyl, arylsulfinyl and arylsulfonyl; wherein each of $R^4$ through $R^9$ is independently selected from hydrido, hydroxy, alkyl, cycloalkyl, cycloalkylalkyl, aralkyl, aryl, alkoxy, aryloxy, aralkoxy, alkoxyalkyl, haloalkyl, hydroxyalkyl, cyano, amino, monoalkylamino, dialkylamino, carboxy, carboxyalkyl, alkanoyl, alkenyl and alkynyl; wherein $R^4$ and $R^5$ may be taken together to form oxo; wherein $R^6$ and $R^7$ may be taken together to form oxo; wherein $R^8$ and $R^9$ may be taken together to form oxo; wherein m is a number selected from one through five; wherein n is a number selected from four through nine; wherein p is a number selected from zero through five; wherein A is selected from aryl, heteroaryl, aryloxy, heteroaryloxy, aralkoxy, heteroaralkoxy, arylamino, heteroarylamino, aralkylamino, heteroaralkylamino, arylthio, heteroarylthio, aralkylthio and heteroaralkylthio; wherein any of the foregoing A groups can be further substituted with one or more substituents independently selected from hydrido, hydroxy, alkyl, cycloalkyl, cycloalkylalkyl, aralkyl, aryl, alkoxy, aryloxy, aralkoxy, alkoxyalkyl, halo, haloalkyl, hydroxyalkyl, cyano, amino, monoalkylamino, dialkylamino, carboxy, carboxyalkyl, alkanoyl, alkenyl and alkynyl; or a pharmaceutically-acceptable salt thereof.

2. Compound of Claim 1 wherein $R^1$ is selected from hydrido, alkyl, cycloalkyl, hydroxyalkyl, haloalkyl, cycloalkylalkyl, alkoxyalkyl, aralkyl, aryl, alkenylalkyl, alkynylalkyl and carboxyalkyl; wherein each of $R^2$ and $R^3$ is independently selected from hydrido, alkyl, cycloalkyl, hydroxyalkyl, haloalkyl, cycloalkylalkyl, alkoxyalkyl, aralkyl, aryl, alkenyl, alkynyl, alkenylalkyl, alkynylalkyl, carboxyalkyl, alkanoyl, alkoxycarbonyl, carboxy and cyanoalkyl; wherein each of $R^4$ through $R^9$ is independently selected from hydrido, hydroxy, alkyl, cycloalkyl, cycloalkylalkyl, aralkyl, aryl, alkoxy, aryloxy, aralkoxy, alkoxyalkyl, haloalkyl, hydroxyalkyl, cyano, amino, monoalkylamino, dialkylamino, carboxy, carboxyalkyl, alkanoyl, alkenyl and alkynyl; wherein $R^4$ and $R^5$ may be taken together to form oxo; wherein m is a number selected from one through four; wherein p is a number selected from zero through four;

wherein A is selected from aryl, heteroaryl, aryloxy, heteroaryloxy, aralkoxy, heteroaralkoxy, arylamino, heteroarylamino, aralkylamino, heteroaralkylamino, arylthio, heteroarylthio, aralkylthio and heteroaralkylthio; wherein any of the foregoing A groups can be further substituted with one or more substituents independently selected from hydrido, hydroxy, alkyl, cycloalkyl, cycloalkylalkyl, aralkyl, aryl, alkoxy, aryloxy, aralkoxy, alkoxyalkyl, halo, haloalkyl, hydroxyalkyl, cyano, amino, monoalkylamino, dialkylamino, carboxy, carboxyalkyl, alkanoyl, alkenyl and alkynyl; or a pharmaceutically acceptable salt thereof.

3. Compound of Claim 2 wherein $R^1$ is selected from hydrido, loweralkyl, cycloalkyl of three to about eight carbon atoms, hydroxyloweralkyl, haloloweralkyl, cycloalkylalkyl of four to about eight carbon atoms,

loweralkoxyloweralkyl, phenylloweralkyl, phenyl, loweralkenylloweralkyl, loweralkynylloweralkyl and carboxyloweralkyl; wherein each of $R^2$ and $R^3$ is independently selected from hydrido, loweralkyl, cycloalkyl of three to about eight carbon atoms, hydroxyloweralkyl, haloloweralkyl, cycloalkylalkyl of four to about eight carbon atoms, loweralkoxyloweralkyl, phenylloweralkyl, phenyl, loweralkenyl, loweralkynyl, loweralkenylloweralkyl, loweralkynylloweralkyl, carboxyloweralkyl, loweralkanoyl, loweralkoxycarbonyl, carboxy and cyanoloweralkyl; wherein each of $R^4$ through $R^9$ is independently selected from hydrido, hydroxy, loweralkyl, cycloalkyl of three to about eight carbon atoms, cycloalkylalkyl of four to about eight carbon atoms, phenylloweralkyl, phenyl, loweralkoxy, phenoxy, phenylloweralkoxy, loweralkoxyloweralkyl, haloloweralkyl, hydroxyloweralkyl, cyano, amino, monoloweralkylamino, diloweralkylamino, carboxy, carboxyloweralkyl, loweralkanoyl, loweralkenyl and loweralkynyl; wherein $R^4$ and $R^5$ may be taken together to form oxo; wherein m is a number selected from one through four; wherein n is a number selected from four through eight; wherein p is a number selected from zero through three; wherein A is selected from phenyl, naphthyl, heteroaryl, phenoxy, naphthyloxy, heteroaryloxy, phenylloweralkoxy, naphthylloweralkoxy, heteroarylloweralkoxy, phenylamino, naphthylamino, heteroarylamino, phenylloweralkylamino, naphthylloweralkylamino, heteroaralkylamino, phenylthio, naphthylthio, heteroarylthio, phenylloweralkylthio and heteroarylloweralkylthio; wherein any of the foregoing A groups can be further substituted with one or more substituents independently selected from hydrido, hydroxy, loweralkyl, cycloalkyl of three to about eight carbon atoms, cycloalkylalkyl of four to about eight carbon atoms, phenylloweralkyl, phenyl, loweralkoxy, phenoxy, phenyloweralkoxy, loweralkoxyloweralkyl, halo, haloloweralkyl, hydroxyloweralkyl, cyano, amino, monoloweralkylamino, diloweralkylamino, carboxy, carboxyloweralkyl, loweralkanoyl, loweralkenyl and loweralkynyl; or a pharmaceutically acceptable salt thereof.

**4.** Compound of Claim 3 wherein $R^1$ is selected from hydrido, loweralkyl, cycloalkyl of three to about eight carbon atoms, cycloalkylalkyl of four to about eight carbon atoms, benzyl, phenyl, loweralkenylloweralkyl and loweralkynylloweralkyl; wherein each of $R^2$ and $R^3$ is independently selected from hydrido, loweralkyl, cycloalkyl of three to about eight carbon atoms, cycloalkylalkyl of four to about eight carbon atoms, benzyl, phenyl, loweralkenyl, loweralkynyl, loweralkenylloweralkyl, loweralkynylloweralkyl, loweralkanoyl and loweralkoxycarbonyl; wherein each of $R^4$ through $R^9$ is independently selected from hydrido, hydroxy, loweralkyl, cycloalkyl of three to about eight carbon atoms, cycloalkylalkyl of four to about eight carbon atoms, benzyl, phenyl, loweralkoxy, phenoxy, benzyloxy, loweralkoxyloweralkyl, haloloweralkyl, hydroxyloweralkyl, loweralkanoyl, loweralkenyl and loweralkynyl; wherein $R^4$ and $R^5$ may be taken together to form oxo; wherein m is a number selected from one through three; wherein n is a number selected from four through seven; wherein p is a number selected from zero through three; wherein A is selected from phenyl, naphthyl, thienyl, phenoxy, benzyloxy, naphthyloxy, thiophenoxy, phenylamino, benzylamino, naphthylamino, phenylthio, benzylthio and naphthylthio; wherein any of the foregoing A groups can be further substituted with one or more substituents independently selected from hydrido, hydroxy, loweralkyl, cycloalkyl of three to about eight carbon atoms, cycloalkylalkyl of four to about eight carbon atoms, loweralkoxy, loweralkoxyloweralkyl, halo, haloloweralkyl, hydroxyloweralkyl, amino, monoloweralkylamino, diloweralkylamino, loweralkanoyl, loweralkenyl and loweralkynyl; or a pharmaceutically acceptable salt thereof.

**5.** Compound of Claim 4 wherein $R^1$ is selected from hydrido, loweralkyl, cycloalkylalkyl of four to six carbon atoms and loweralkenylloweralkyl; wherein each of $R^2$ and $R^3$ is independently selected from hydrido and loweralkyl; wherein each of $R^4$ through $R^9$ is independently selected from hydrido, hydroxy, loweralkyl, benzyl, phenoxy, benzyloxy and haloloweralkyl; wherein m is a number selected from one through three; wherein n is a number selected from four through six; wherein p is a number selected from zero through two; wherein A is selected from phenyl, naphthyl and thienyl; wherein any of the foregoing A groups can be further substituted with one or more substituents independently selected from hydrido, hydroxy, loweralkyl, loweralkoxy, halo, haloloweralkyl, amino, monoloweralkylamino and diloweralkylamino; or a pharmaceutically acceptable salt thereof.

**6.** Compound of Claim 5 wherein $R^1$ is selected from hydrido, methyl, ethyl, propyl, cyclopropylmethyl, allyl and dimethylallyl; wherein each of $R^2$ and $R^3$ is independently selected from hydrido, methyl, ethyl and propyl; wherein each of $R^4$ through $R^9$ is independently selected from hydrido, hydroxy, methyl, ethyl, propyl, benzyl, phenoxy, benzyloxy and haloloweralkyl; wherein m is a number selected from one through three; wherein n is a number selected from four through six; wherein p is a number selected from zero or one; wherein A is phenyl or naphthyl; wherein any of the foregoing A groups can be

further substituted with one or more substituents independently selected from hydroxy, methyl, ethyl, propyl, methoxy, ethoxy, methylenedioxy, halo, trifluoromethyl, amino, methylamino and dimethylamino; or a pharmaceutically acceptable salt thereof.

**7.** Compound of Claim 6 which is N-[2-(3,4-dichlorophenyl)ethyl]-N-methyl-2-(1-pyrrolidinyl)ethylamine or a pharmaceutically acceptable salt thereof.

**8.** A pharmaceutical composition comprising a therapeutically-effective amount of an active compound for treating or preventing a CNS-related disorder and a pharmaceutically-acceptable carrier or diluent, said active compound selected from a family of compounds of the formula

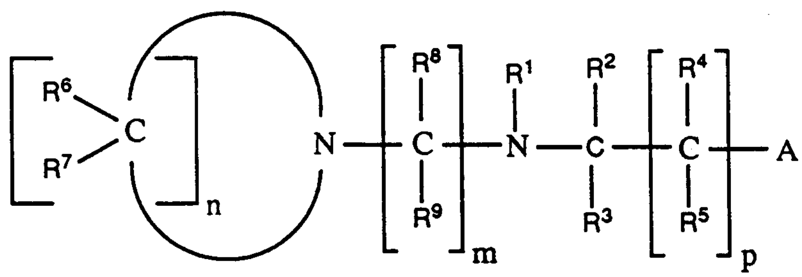

(I)

wherein $R^1$ is selected from hydrido, alkyl, cycloalkyl, hydroxyalkyl, haloalkyl, cycloalkylalkyl, alkoxyalkyl, aralkyl, aryl, alkenylalkyl, alkynylalkyl, carboxyalkyl, alkanoyl, alkylsulfinyl, alkylsulfonyl, arylsulfinyl and arylsulfonyl; wherein each of $R^2$ and $R^3$ is independently selected from hydrido, alkyl, cycloalkyl, hydroxyalkyl, haloalkyl, cycloalkylalkyl, alkoxyalkyl, aralkyl, aryl, alkenyl, alkynyl, alkenylalkyl, alkynylalkyl, carboxyalkyl, alkanoyl, alkoxycarbonyl, carboxy, cyanoalkyl, alkylsulfinyl, alkylsulfonyl, arylsulfinyl and arylsulfonyl; wherein each of $R^4$ through $R^9$ is independently selected from hydrido, hydroxy, alkyl, cycloalkyl, cycloalkylalkyl, aralkyl, aryl, alkoxy, aryloxy, aralkoxy, alkoxyalkyl, haloalkyl, hydroxyalkyl, cyano, amino, monoalkylamino, dialkylamino, carboxy, carboxyalkyl, alkanoyl, alkenyl and alkynyl; wherein $R^4$ and $R^5$ may be taken together to form oxo; wherein $R^6$ and $R^7$ may be taken together to form oxo; wherein $R^8$ and $R^9$ may be taken together to form oxo; wherein m is a number selected from one through five; wherein n is a number selected from four through nine; wherein p is a number selected from zero through five; wherein A is selected from aryl, heteroaryl, aryloxy, heteroaryloxy, aralkoxy, heteroaralkoxy, arylamino, heteroarylamino, aralkylamino, heteroaralkylamino, arylthio, heteroarylthio, aralkylthio and heteroaralkylthio; wherein any of the foregoing A groups can be further substituted with one or more substituents independently selected from hydrido, hydroxy, alkyl, cycloalkyl, cycloalkylalkyl, aralkyl, aryl, alkoxy, aryloxy, aralkoxy, alkoxyalkyl, halo, haloalkyl, hydroxyalkyl, cyano, amino, monoalkylamino, dialkylamino, carboxy, carboxyalkyl, alkanoyl, alkenyl and alkynyl; or a pharmaceutically-acceptable salt thereof.

**9.** The composition of Claim 8 wherein $R^1$ is selected from hydrido, alkyl, cycloalkyl, hydroxyalkyl, haloalkyl, cycloalkylalkyl, alkoxyalkyl, aralkyl, aryl, alkenylalkyl, alkynylalkyl and carboxyalkyl; wherein each of $R^2$ and $R^3$ is independently selected from hydrido, alkyl, cycloalkyl, hydroxyalkyl, haloalkyl, cycloalkylalkyl, alkoxyalkyl, aralkyl, aryl, alkenyl, alkynyl, alkenylalkyl, alkynylalkyl, carboxyalkyl, alkanoyl, alkoxycarbonyl, carboxy and cyanoalkyl; wherein each of $R^4$ through $R^9$ is independently selected from hydrido, hydroxy, alkyl, cycloalkyl, cycloalkylalkyl, aralkyl, aryl, alkoxy, aryloxy, aralkoxy, alkoxyalkyl, haloalkyl, hydroxyalkyl, cyano, amino, monoalkylamino, dialkylamino, carboxy, carboxyalkyl, alkanoyl, alkenyl and alkynyl; wherein $R^4$ and $R^5$ may be taken together to form oxo; wherein m is a number selected from one through four; wherein p is a number selected from zero through four;

wherein A is selected from aryl, heteroaryl, aryloxy, heteroaryloxy, aralkoxy, heteroaralkoxy, arylamino, heteroarylamino, aralkylamino, heteroaralkylamino, arylthio, heteroarylthio, aralkylthio and heteroaralkylthio; wherein any of the foregoing A groups can be further substituted with one or more substituents independently selected from hydrido, hydroxy, alkyl, cycloalkyl, cycloalkylalkyl, aralkyl, aryl, alkoxy, aryloxy, aralkoxy, alkoxyalkyl, halo, haloalkyl, hydroxyalkyl, cyano, amino, monoalkylamino, dialkylamino, carboxy, carboxyalkyl, alkanoyl, alkenyl and alkynyl; or a pharmaceutically acceptable salt thereof.

**10.** The composition of Claim 9 wherein $R^1$ is selected from hydrido, loweralkyl, cycloalkyl of three to about eight carbon atoms, hydroxyloweralkyl, haloloweralkyl, cycloalkylalkyl of four to about eight carbon atoms, loweralkoxyloweralkyl, phenylloweralkyl, phenyl, loweralkenylloweralkyl, loweralkynylloweralkyl and carboxyloweralkyl; wherein each of $R^2$ and $R^3$ is independently selected from hydrido, loweralkyl, cycloalkyl of three to about eight carbon atoms, hydroxyloweralkyl, haloloweralkyl, cycloalkylalkyl of four to about eight carbon atoms, loweralkoxyloweralkyl, phenylloweralkyl, phenyl, loweralkenyl, loweralkynyl, loweralkenylloweralkyl, loweralkynylloweralkyl, carboxyloweralkyl, loweralkanoyl, loweralkoxycarbonyl, carboxy and cyanoloweralkyl; wherein each of $R^4$ through $R^9$ is independently selected from hydrido, hydroxy, loweralkyl, cycloalkyl of three to about eight carbon atoms, cycloalkylalkyl of four to about eight carbon atoms, phenylloweralkyl, phenyl, loweralkoxy, phenoxy, phenylloweralkoxy, loweralkoxyloweralkyl, haloloweralkyl, hydroxyloweralkyl, cyano, amino, monoloweralkylamino, diloweralkylamino, carboxy, carboxyloweralkyl, loweralkanoyl, loweralkenyl and loweralkynyl; wherein $R^4$ and $R^5$ may be taken together to form oxo; wherein m is a number selected from one through four; wherein n is a number selected from four through eight; wherein p is a number selected from zero through three; wherein A is selected from phenyl, naphthyl, heteroaryl, phenoxy, naphthyloxy, heteroaryloxy, phenylloweralkoxy, naphthylloweralkoxy, heteroarylloweralkoxy, phenylamino, naphthylamino, heteroarylamino, phenylloweralkylamino, naphthylloweralkylamino, heteroaralkylamino, phenylthio, naphthylthio, heteroarylthio, phenylloweralkylthio and heteroarylloweralkylthio; wherein any of the foregoing A groups can be further substituted with one or more substituents independently selected from hydrido, hydroxy, loweralkyl, cycloalkyl of three to about eight carbon atoms, cycloalkylalkyl of four to about eight carbon atoms, phenylloweralkyl, phenyl, loweralkoxy, phenoxy, phenyloweralkoxy, loweralkoxyloweralkyl, halo, haloloweralkyl, hydroxyloweralkyl, cyano, amino, monoloweralkylamino, diloweralkylamino, carboxy, carboxyloweralkyl, loweralkanoyl, loweralkenyl and loweralkynyl; or a pharmaceutically acceptable salt thereof.

**11.** The composition of Claim 10 wherein $R^1$ is selected from hydrido, loweralkyl, cycloalkyl of three to about eight carbon atoms, cycloalkylalkyl of four to about eight carbon atoms, benzyl, phenyl, loweralkenylloweralkyl and loweralkynylloweralkyl; wherein each of $R^2$ and $R^3$ is independently selected from hydrido, loweralkyl, cycloalkyl of three to about eight carbon atoms, cycloalkylalkyl of four to about eight carbon atoms, benzyl, phenyl, loweralkenyl, loweralkynyl, loweralkenylloweralkyl, loweralkynylloweralkyl, loweralkanoyl and loweralkoxycarbonyl; wherein each of $R^4$ through $R^9$ is independently selected from hydrido, hydroxy, loweralkyl, cycloalkyl of three to about eight carbon atoms, cycloalkylalkyl of four to about eight carbon atoms, benzyl, phenyl, loweralkoxy, phenoxy, benzyloxy, loweralkoxyloweralkyl, haloloweralkyl, hydroxyloweralkyl, loweralkanoyl, loweralkenyl and loweralkynyl; wherein $R^4$ and $R^5$ may be taken together to form oxo; wherein m is a number selected from one through three; wherein n is a number selected from four through seven; wherein p is a number selected from zero through three; wherein A is selected from phenyl, naphthyl, thienyl, phenoxy, benzyloxy, naphthyloxy, thiophenoxy, phenylamino, benzylamino, naphthylamino, phenylthio, benzylthio and naphthylthio; wherein any of the foregoing A groups can be further substituted with one or more substituents independently selected from hydrido, hydroxy, loweralkyl, cycloalkyl of three to about eight carbon atoms, cycloalkylalkyl of four to about eight carbon atoms, loweralkoxy, loweralkoxyloweralkyl, halo, haloloweralkyl, hydroxyloweralkyl, amino, monoloweralkylamino, diloweralkylamino, loweralkanoyl, loweralkenyl and loweralkynyl; or a pharmaceutically acceptable salt thereof.

**12.** The composition of Claim 11 wherein $R^1$ is selected from hydrido, loweralkyl, cycloalkylalkyl of four to six carbon atoms and loweralkenylloweralkyl; wherein each of $R^2$ and $R^3$ is independently selected from hydrido and loweralkyl; wherein each of $R^4$ through $R^9$ is independently selected from hydrido, hydroxy, loweralkyl, benzyl, phenoxy, benzyloxy and haloloweralkyl; wherein m is a number selected from one through three; wherein n is a number selected from four through six; wherein p is a number selected from zero through two; wherein A is selected from phenyl, naphthyl and thienyl; wherein any of the foregoing A groups can be further substituted with one or more substituents independently selected from hydrido, hydroxy, loweralkyl, loweralkoxy, halo, haloloweralkyl, amino, monoloweralkylamino and diloweralkylamino; or a pharmaceutically acceptable salt thereof.

**13.** The composition of Claim 12 wherein $R^1$ is selected from hydrido, methyl, ethyl, propyl, cyclopropylmethyl, allyl and dimethylallyl; wherein each of $R^2$ and $R^3$ is independently selected from hydrido, methyl, ethyl and propyl; wherein each of $R^4$ through $R^9$ is independently selected from hydrido, hydroxy, methyl, ethyl, propyl, benzyl, phenoxy, benzyloxy and haloloweralkyl; wherein m is a number

selected from one through three; wherein n is a number selected from four through six; wherein p is a number selected from zero or one; wherein A is phenyl or naphthyl; wherein any of the foregoing A groups can be further substituted with one or more substituents independently selected from hydroxy, methyl, ethyl, propyl, methoxy, ethoxy, methylenedioxy, halo, trifluoromethyl, amino, methylamino and dimethylamino; or a pharmaceutically acceptable salt thereof.

**14.** The composition of Claim 13 wherein said active compound is N-[2-(3,4-dichlorophenyl)ethyl]-N-methyl-2-(1-pyrrolidinyl)ethylamine or a pharmaceutically acceptable salt thereof.

**15.** Use of a compound according to any of claims 1 to 6 for preparing a medicament for treating a CNS-related disorder in a patient.

**16.** Use according to Claim 15 wherein said compound is N-[2-(3,4-dichlorophenyl)ethyl]-N-methyl-2-(1-pyrrolidinyl)ethylamine or a pharmaceutically acceptable salt thereof.

**17.** Use according to Claim 15 wherein said CNS-related disorder is cerebral ischemia.

**18.** Use according to Claim 15 wherein said CNS-related disorder is a psychotic disorder.

**19.** Use according to Claim 15 wherein said CNS-related disorder is a convulsive disorder.

**20.** Use according to Claim 15 wherein said CNS-related disorder is Parkinsonism.